# EUROPEAN PATENT APPLICATION

(11) **EP 3 111 822 A1**
(43) Date of publication of application: **04.01.2017**
(21) Application number: 15776796.3
(22) Date of filing: 27.03.2015
(51) Int. Cl.: A61B 1/00, A61B 1/04, A61B 1/06, H04N 7/18

(54) **FLUORESCENCE ENDOSCOPY SYSTEM**

(30) Priority: 08.04.2014 JP 2014079764
(71) Applicant: OLYMPUS CORPORATION, Hachioji-shi Tokyo 192-8507 (JP)
(72) Inventor: TAKEUCHI, Yuichi, Tokyo 192-8507 (JP); WATANABE, Toshiaki, Tokyo 192-8507 (JP)
(74) Representative: Gunzelmann, Rainer
(86) International application number: PCT/JP2015/059654
(87) International publication number: WO 2015/156153

(57) **Abstract**

A fluorescence observation endoscope system includes a light source apparatus configured to be able to simultaneously emit light in a first wavelength band as fluorescence which is radiated onto medicine administered to a living body to thereby emit fluorescence, light in a second wavelength band and light in a third wavelength band which are visible light, an image pickup section configured to include an image pickup device to simultaneously receive the fluorescence and reflected light of the light in the second and third wavelength bands, and a signal processing apparatus configured to generate color display images from an image pickup signal of the fluorescence acquired by the image pickup section and second and third image pickup signals acquired from the reflected light of the light in the second and third wavelength bands to be displayed in different colors.

## Description

### Technical Field

The present invention relates to a fluorescence observation endoscope system that performs fluorescence observation.

### Background Art

In recent years, endoscopes have been widely used in a medical field or the like. In addition to normal light observation (normal observation) where observation is performed using light in a visible wavelength band as normal light, there are also cases where fluorescence observation is performed by administering medicine to a living body region to be examined and receiving fluorescence generated.

For example, a related art described in Japanese Patent Application Laid-Open Publication No. 2011-167337 discloses a light source apparatus that simultaneously radiates visible light such as blue, green and yellow light, and near-infrared light (ICG excitation light), an image pickup unit provided with a plurality of image pickup devices that receive reflected light of each color and fluorescence generated by excitation light respectively, and a signal processing apparatus that generates a display image from a signal by the image pickup unit.

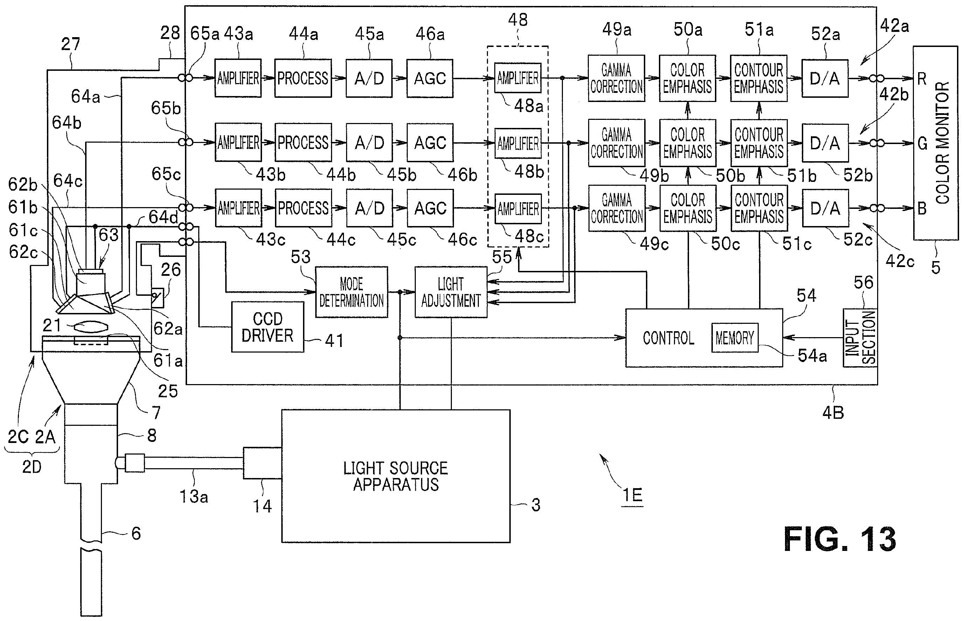

The above-described related art discloses, as shown in Figure 13 in the publication, contents of displaying a normal light image (normal image) and one ICG fluorescence image respectively and displaying a synthesized image obtained by synthesizing the normal light image and an image calculated from a difference value between two different fluorescence images.

However, the above-described related art does not disclose details of the color display in the display image when displaying the synthesized image obtained by synthesizing the normal light image and the image calculated from the difference value between the fluorescence images. When a fluorescence image is displayed, contours and color tones of an organ or a biological tissue appear too complicated to grasp using fluorescence images alone, and therefore when treatment using a treatment instrument is performed, it is desirable to display the images of an organ or a biological tissue with differences in contours and color tones reflected.

For example, based on contours alone, it is difficult to distinguish between a fat-rich tissue and a membrane-shaped tissue having different color tones, and it is therefore desirable to enable those tissues to be displayed by reflecting the differences in color tones. The above-described related art generates a synthesized image from a normal light image and an image calculated from a difference value between fluorescence images, but since the related art does not disclose contents of how the image is displayed in color at all, the related art does not meet the requirements for displaying the images by reflecting differences in the contours and color tones of an organ or a biological tissue.

The present invention has been implemented in view of the aforementioned problems and it is an object of the present invention to provide a fluorescence observation endoscope system capable of generating images that reflect differences in contours and color tones between a fluorescence image and a biological tissue.

### Disclosure of Invention

### Means for Solving the Problem

A fluorescence observation endoscope system according to an aspect of the present invention includes a light source apparatus configured to be able to simultaneously emit light in a first wavelength band which is radiated onto medicine administered to a living body to thereby emit fluorescence, light in a second wavelength band which is visible light having a wavelength band different from the first wavelength band and light in a third wavelength band which is visible light having a wavelength band different from the first and second wavelength bands, an image pickup section configured to include an image pickup device to simultaneously receive the fluorescence and reflected light of the light in the second and third wavelength bands, and a signal processing apparatus configured to perform image processing of generating color display images from an image pickup signal of the fluorescence acquired by the image pickup section and second and third image pickup signals acquired from the reflected light of the light in the second and third wavelength bands to be displayed in different colors.

### Brief Description of the Drawings

Fig. 1 is a diagram illustrating an overall configuration of a fluorescence observation endoscope system according to a first embodiment of the present invention;
Fig. 2 is a diagram illustrating the fluorescence observation endoscope system showing an internal configuration of an endoscope and a video processor or the like in Fig. 1;
Fig. 3A is a diagram illustrating a wavelength band of illuminating light emitted when the light source apparatus is in a fluorescence observation mode;
Fig. 3B is a diagram illustrating a wavelength band of illuminating light emitted when the light source apparatus is in a normal-light observation mode;
Fig. 4 is a diagram illustrating a transmission characteristic of a light-receiving filter provided in an image pickup section and a range of a wavelength band shielded by an excitation light cut filter;
Fig. 5 is a flowchart illustrating typical operation contents according to the first embodiment of the present invention;
Fig. 6 is a diagram illustrating an overall configuration of a fluorescence observation endoscope system according to a first modification of the first embodiment;
Fig. 7 is a diagram illustrating a wavelength band of illuminating light emitted when the light source apparatus according to the first modification is in a fluorescence observation mode or the like;
Fig. 8 is a diagram illustrating a transmission characteristic of a light-receiving filter provided in the image pickup section according to the first modification and a range of a wavelength band shielded by an excitation light cut filter;
Fig. 9 is a diagram illustrating an overall configuration of a fluorescence observation endoscope system according to a second modification of the first embodiment;
Fig. 10 is a diagram illustrating a wavelength band of illuminating light emitted by the light source apparatus in a fluorescence observation mode according to the second modification;
Fig. 11 is a diagram illustrating a transmission characteristic of a light-receiving filter of the image pickup section according to the second modification;
Fig. 12A is a diagram illustrating an overall configuration of a fluorescence observation endoscope system according to a third modification of the first embodiment;
Fig. 12B is a diagram illustrating a light source apparatus according to a fourth modification of the first embodiment;
Fig. 13 is a diagram illustrating an overall configuration of a fluorescence observation endoscope system according to a second embodiment of the present invention;
Fig. 14 is a diagram illustrating a transmission characteristic of a dichroic prism provided in the image pickup section;
Fig. 15 is a diagram illustrating an overall configuration of a fluorescence observation endoscope system according to a first modification of the second embodiment of the present invention;
Fig. 16A is a diagram illustrating a transmission characteristic of a dichroic prism provided in the image pickup section according to the first modification and a range of a wavelength band shielded by an excitation light cut filter;
Fig. 16B is a diagram illustrating a transmission characteristic of the excitation filter provided in the light source apparatus according to the first modification;
Fig. 17 is a diagram illustrating a relationship among a plurality of types of auto fluorescence substances, and corresponding excitation wavelength and fluorescence wavelength in a table format;
Fig. 18 is a diagram illustrating an overall configuration of a fluorescence observation endoscope system according to a second modification of the second embodiment of the present invention;
Fig. 19A is a diagram illustrating a transmission characteristic of a dichroic prism provided in the image pickup section and a range of a wavelength band shielded by an excitation light cut filter; and
Fig. 19B is a diagram illustrating a wavelength band of illuminating light emitted by the light source apparatus via an excitation filter in the case of a fluorescence observation mode.

### Best Mode for Carrying Out the Invention

Hereinafter, embodiments of the present invention will be described with reference to the accompanying drawings.

### (First Embodiment)

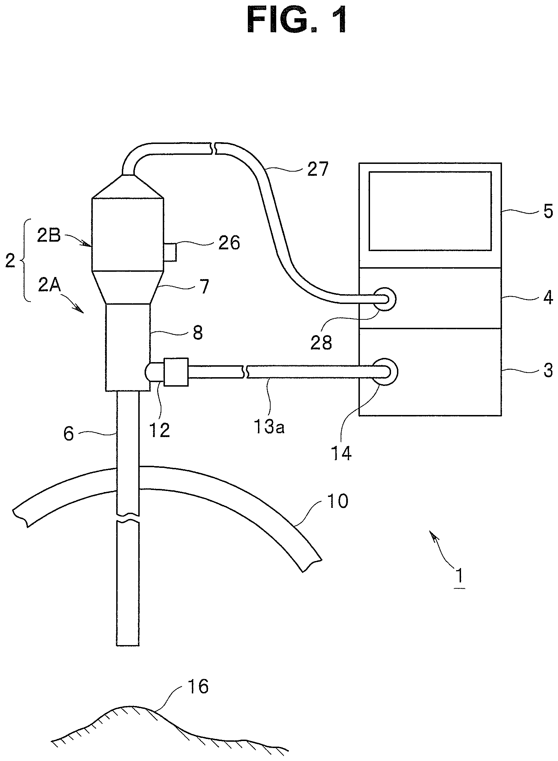

As shown in Fig. 1, a fluorescence observation endoscope system 1 according to a first embodiment of the present invention includes an endoscope 2 configured to be inserted into a subject such as an abdomen 10, pick up an image of the object such as a biological tissue in the subject and output the image as an image pickup signal, a light source apparatus 3 configured to emit illuminating light for illuminating the object to the endoscope 2, a video processor 4 as a signal processing apparatus configured to drive an image pickup section (or an image pickup apparatus) incorporated in the endoscope 2, perform signal processing on the image pickup signal outputted from the endoscope 2 and output the processed signal as an image signal (video signal), and a color monitor 5 as a color display apparatus configured to display an image of the object based on the image signal outputted from the video processor 4.

The endoscope 2 shown in Fig. 1 is constructed of an optical endoscope 2A including an elongated insertion portion 6 and a television camera 2B attached to an eyepiece section 7 of the optical endoscope 2A and configured to incorporate an image pickup device. Note that the endoscope is not limited to the endoscope 2 composed of the optical endoscope 2A and the television camera 2B attached to the optical endoscope 2A shown in Fig. 1, but the endoscope may also be an electronic endoscope with an image pickup device disposed at a distal end of the insertion portion.

The endoscope 2 includes, for example, the elongated insertion portion 6 inserted into the abdomen 10 of a patient, a grasping portion 8 provided at a rear end (proximal end) of the insertion portion 6 and the eyepiece section 7 provided at a rear end of the grasping portion 8.

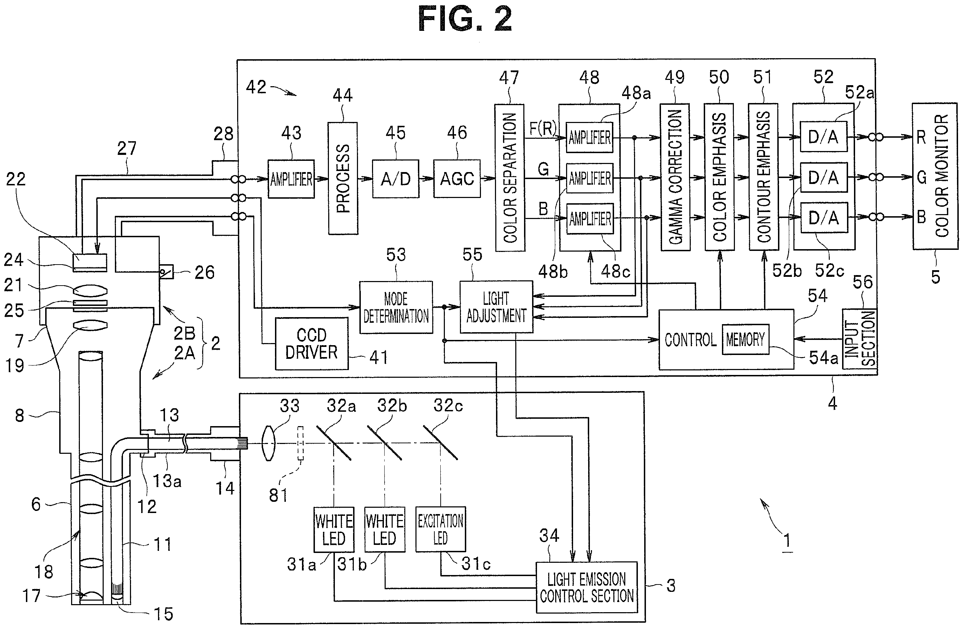

As shown in Fig. 2, a light guide 11 as an illuminating light transmitting member configured to transmit illuminating light emitted from the light source apparatus 3 is inserted into the insertion portion 6 and a rear end of the light guide 11 reaches a light guide pipe sleeve 12 near the grasping portion 8. One end of a cable 13a through which a light guide 13 is inserted is connected to the light guide pipe sleeve 12 and a light guide connector 14 at the other end is detachably connected to the light source apparatus 3.

The light source apparatus 3 emits (generates) illuminating light for fluorescence observation and illuminating light for normal light observation according to an observation mode as will be described later. The illuminating light of the light source apparatus 3 passes through the light guide 13 and the light guide 11, and is emitted from a distal end of the light guide 11 to the living body (tissue) side which is an observation target such as an affected area 16 in the body cavity (see Fig. 1) via an illumination lens 15. Note that the illumination lens 15 is provided in an illuminating window provided on a distal end face of the insertion portion 6.

An observation window is provided adjacent to the illuminating window, an objective lens 17 is disposed in the observation window and the objective lens 17 forms an optical image of light from the object side on the illuminated affected area 16 side.

The optical image formed by the objective lens 17 is transmitted toward the rear side through a relay lens system 18 as optical image transmitting member disposed from the interior of the insertion portion 6 to the vicinity of the eyepiece section 7. Note that the optical image transmitting member may also be formed using an image guide formed of a fiber bundle instead of the relay lens system 18.

An eyepiece lens 19 is disposed in the eyepiece section 7, and in the case of an optical image of a visible wavelength region, the user such as an operator can observe the optical image by the naked eye via the eyepiece lens 19.

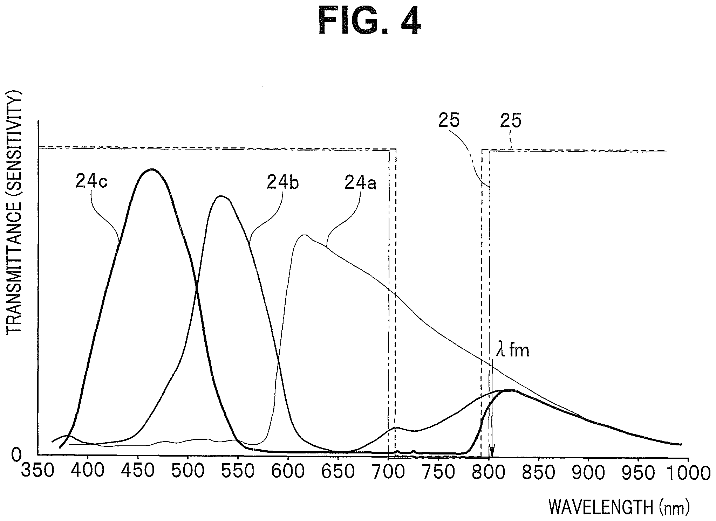

An image forming lens 21 and, for example, a charge coupled device (abbreviated as a "CCD") 22 as an image pickup device making up an image pickup section whose image pickup surface is disposed at an image forming position of the image forming lens 21 are provided inside the television camera 2B attached to the eyepiece section 7. A mosaic filter 24 provided with an R filter 24a, a G filter 24b and a B filter 24c (see Fig. 4) as color filters that transmit light in red (R), green (G) and blue (B) wavelength bands respectively is disposed for each pixel forming an image pickup surface of the CCD 22 immediately ahead of the image pickup surface of the CCD 22. Note that the image pickup section (or image pickup apparatus) may be defined as having an image pickup device or may be defined as including an optical system such as the image forming lens 21 configured to form an optical image on the image pickup surface of the image pickup device in addition to the image pickup device.

Fig. 4 shows an example of transmission characteristics of the R filter 24a, G filter 24b and B filter 24c making up the mosaic filter 24.

Therefore, as will be described later, when the light source apparatus 3 is in a normal light observation (normal observation) mode in which the light source apparatus 3 emits illuminating light in a visible wavelength band as the illuminating light, the light source apparatus 3 color-separates light reflected on the object side and made incident according to the transmission characteristics of the R filter 24a, the G filter 24b and the B filter 24c shown in Fig. 4 and guides the light to pixels on the image pickup surface. Note that when the image pickup section is defined as an image pickup device provided with the R filter 24a, the G filter 24b and the B filter 24c, the transmission characteristics in Fig. 4 can be regarded as sensitivity of pixels that receive light transmitting through the R filter 24a, the G filter 24b and the B filter 24c of the image pickup device respectively. For this reason, in Fig. 4, the vertical axis is represented as transmittance (sensitivity).

Furthermore, in the present embodiment, in order to be able to perform the above-described fluorescence observation, an excitation light cut filter 25 is disposed closer to the incident light side than the image pickup surface of the CCD 22 as a cut filter configured to cut the reflected light reflected on the object side when illuminating light (of excitation light) is radiated so as to prevent the reflected light from entering pixels that receive fluorescence (pixels that receive the light transmitting through the R filter 24a in the case of the present embodiment). In Fig. 2, the excitation light cut filter 25 is disposed, for example, in front of the image forming lens 21, but the position of the excitation light cut filter 25 is not limited to this position.

In the present embodiment, indocyanine green (abbreviated as "ICG") is assumed to be used as the fluorescent agent (also simply called "medicine")used for fluorescence observation. The ICG is administered to the living body (tissue) to be observed, the ICG is irradiated with excitation light and the fluorescence emitted from the excited ICG is imaged using the CCD 22 provided with the mosaic filter 24.

The ICG exhibits a fluorescence generation characteristic in which intensity of the fluorescence reaches a local maximum at a wavelength of λfm (λfm=805 nm) shown in Fig. 4. It is known that the ICG has a characteristic in which absorption reaches a local maximum at a wavelength of λex (λex=774 nm) which is a little shorter than wavelength λfm.

For this reason, the excitation light cut filter 25 according to the present embodiment cuts the wavelength band range of excitation light that excites the ICG and sets the characteristic to such a characteristic in which the light passes through the wavelength band of the fluorescence which reaches a peak at the wavelength λfm. In Fig. 4, a dotted line shows a light-shielding range (light-shielded wavelength band) by the excitation light cut filter 25 in terms of transmittance. More specifically, light in a wavelength band of 710 nm to 790 nm is fully cut (e.g., light-shielded at a transmittance nearly 0%). The excitation light cut filter 25 transmits light in other wavelength bands at a large transmittance.

The fluorescence in a near-infrared wavelength band of 790 nm to 900 nm or in a wavelength band of 790 nm to 850 nm on a long wavelength side relative to a value 790 nm at an end on the long wavelength side in the wavelength band cut by the excitation light cut filter 25 is received using the transmission characteristic on the infrared wavelength band side of the R filter 24a. In the example shown in Fig. 4, the R filter 24a has a characteristic of transmitting the fluorescence further on the long wavelength side beyond 900 nm, whereas the ICG has sufficiently small intensity for generating fluorescence in the vicinity of 850 nm to 900 nm. For this reason, light may be received by cutting the band on the long wavelength side beyond 850 or 900 nm.

As shown in Fig. 2, the television camera 2B is provided with an observation mode changeover switch (which may also be simply referred to as "changeover switch") 26 as observation mode changing means for changing an observation mode. A signal connector 28 provided at an end of a signal cable 27 that extends from the television camera 2B is detachably connected to the video processor 4.

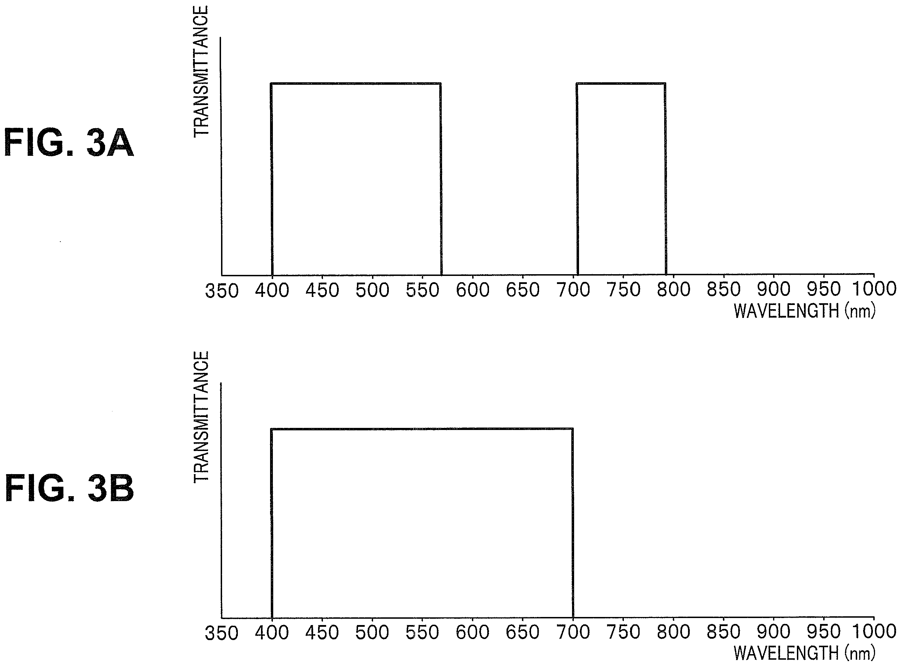

In the present embodiment, when a fluorescence observation mode is selected by the changeover switch 26, the light source apparatus 3 simultaneously emits, as illuminating light, excitation light (light of 710 nm to 790 nm in Fig. 3A) as light in a first wavelength band to generate fluorescence as shown in Fig. 3A, and light in second and third wavelength bands different from each other, which are different from the first wavelength band and included in a visible wavelength band.

Note that the light in the second and third wavelength bands corresponds to light in the G and B wavelength bands as shown in Fig. 3A, which are also expressed as G light and B light respectively. In the fluorescence observation mode, the CCD 22 as the image pickup section picks up an image of the fluorescence using pixels that receive light which passes through the R filter 24a (in other words, pixels provided with the R filter 24a or further simplified and abbreviated as "pixels of the R filter 24a") and picks up an image of reflected light of the G light and the B light using pixels that receive light which passes through the G filter 24b and pixels that receive light which passes through the B filter 24c (abbreviated as "pixels of the G filter 24b" and "pixels of the B filter 24c") to generate reference light images (reflected light images) (to complement fluorescence images).

The video processor 4 generates a fluorescence image signal and two image signals: a reflected light image (or reference light image) from the fluorescence image pickup signal acquired by the CCD 22 as a single image pickup device that forms the image pickup section and the reflected light of the reference light, and the color monitor 5 generates a display image on which the fluorescence image and the reflected light image are displayed in different colors. The present embodiment displays the fluorescence image and images of reflected light in two different wavelength bands in different colors making it possible to display the reflected light image (reference light image) by reflected light that reflects not only contours or structure of a biological tissue which are undiscernible from the fluorescence image but also color tones corresponding to different biological tissues.

Furthermore, when a normal-light observation mode is selected by the changeover switch 26, the light source apparatus 3 does not emit excitation light in the fluorescence observation mode, but emits light in an R wavelength band, that is, R light together with the light in the second wavelength band and the light in the third wavelength band (G light and B light).

As shown in Fig. 2, the light source apparatus 3 is provided with white light-emitting diodes (abbreviated as "white LEDs") 31a and 31 b configured to generate white light, an excitation LED 31c configured to generate (at least) (light including a wavelength band of) excitation light, dichroic mirrors 32a, 32b and 32c as optical elements, a condensing lens 33, and a light emission control section (or light emission control circuit) 34.

The white LED 31a and the dichroic mirror 32a (and the condensing lens 33) simultaneously generate the above-described light in the second and third wavelength band. The white LED 31 a generates white light that covers a visible wavelength band and only the G light and the B light corresponding to the light in the second and third wavelength band of the white light are selectively reflected by the dichroic mirror 32a disposed on an optical path opposite to the white LED 31a and (the G light and the B light) are made incident on an end face of the light guide 13 after passing through the condensing lens 33 disposed on the reflected light path.

The dichroic mirror 32a has a characteristic of selectively reflecting only the G light and the B light as the light in the second and third wavelength band as described above and selectively transmitting light in a longer wavelength band than the G light and B light. Therefore, the white light of the white LED 31 a is band-limited by the dichroic mirror 32a whereby light within a range of wavelength band of 400 nm to 570 nm in Fig. 3A (substantially G light and B light) is emitted onto the light guide 13 side and is further radiated onto the object side via the light guide 11.

The white LED 31b and the dichroic mirror 32b (and the condensing lens 33) generate light other than the above-described light in the second and third wavelength bands in the visible wavelength band, more specifically, light in a wavelength band of 570 nm to 700 nm (substantially R light). Only the R light of the white light of the white LED 31b is selectively reflected by the dichroic mirror 32b disposed on an optical path opposite to the white LED 31b, passes through the dichroic mirror 32a disposed on the reflected light path and is made incident upon the end face of the light guide 13 via the condensing lens 33.

That is, in the case of the normal observation mode, the white LEDs 31a and 31b emit light and white light in the visible wavelength region is made incident upon the end face of the light guide 13.

The excitation LED 31 c generates excitation light near a cut wavelength band which is cut by an excitation light cut filter, and only a light portion within the cut wavelength band of the excitation light is selectively reflected by the dichroic mirror 32c disposed on the light path opposite to the excitation LED 31c, passes through the dichroic mirrors 32b and 32a disposed on the reflected light path and is made incident upon the end face of the light guide 13 via the condensing lens 33.

The dichroic mirror 32c exhibits a characteristic of selectively reflecting only light within the cut wavelength band as described above. Therefore, the excitation light of the excitation LED 31c is band-limited by the dichroic mirror 32c, and light within a range of a wavelength band of, for example, 710 nm to 790 nm in Fig. 3A passes through the dichroic mirrors 32b and 32a as excitation light, is emitted onto the light guide 13 side and radiated onto the object side via the light guide 11. Note that without being limited to the case where excitation light is band-limited by the dichroic mirror 32c, the excitation LED 31c may be configured to generate excitation light within the cut wavelength band.

In the case of the fluorescence observation mode, the light emission control section 34 causes the white LED 31a and the excitation LED 31 c to simultaneously emit light to emit illuminating light in the wavelength band shown in Fig. 3A.

On the other hand, in the case of the normal-light observation mode, the light emission control section 34 causes the white LED 31a and the white LED 31b to simultaneously emit light to emit illuminating light in the visible wavelength band shown in Fig. 3B. Note that as shown by a dotted line in Fig. 2, in the case of the fluorescence observation mode, an excitation filter 81 configured to cut light in a short wavelength of, for example, 450 nm or less including an excitation wavelength at which auto fluorescence is generated or a band limiting filter may be disposed on the illuminating light path to prevent auto fluorescence from mixing into the fluorescence caused by a fluorescent agent to be observed.

As described above, the R filter 24a, the G filter 24b and the B filter 24c making up the mosaic filter 24 of the CCD 22 have the transmission characteristic shown in Fig. 4. For this reason, in the case of the normal-light observation mode, when illuminating light in the visible wavelength band shown in Fig. 3B is radiated onto the object side and the reflected light reflected on the object side is made incident upon the CCD 22, pixels of the R filter 24a receive the R light in the R wavelength band of the reflected light, pixels of the G filter 24b receive the G light in the G wavelength band in the reflected light, pixels of the B filter 24a receive the B light in the B wavelength band of the reflected light. The video processor 4 generates image signals of normal light images as reflected light images of R, G and B from image pickup signals of the CCD 22 that picks up images of reflected light of the R light, the G light and the B light.

On the other hand, in the case of the fluorescence observation mode, when illuminating light composed of the G light and the B light in the G and B wavelength bands and the excitation light which becomes a near-infrared wavelength band shown in Fig. 3A is radiated onto the object side, and the reflected light reflected on the object side and the excitation light and the fluorescence are made incident upon the CCD 22, the excitation light is cut by the excitation light cut filter 25, pixels of the R filter 24a receive the light in the fluorescence wavelength band belonging to the near-infrared wavelength band, pixels of the G filter 24b receive the G light in the G wavelength band and pixels of the B filter 24a receive the B light in the B wavelength band.

Note that when performing fluorescence observation, since the intensity of the fluorescence is much weaker than the intensity of the reflected light (a few percent or less), the fluorescence is susceptible to the reflected light of the excitation light. In the present embodiment, the wavelength band of the excitation light is sufficiently cut by the excitation light cut filter 25 so as to prevent the reflected light of the excitation light from affecting reception of fluorescence.

The characteristic example shown in Fig. 4 shows that when fluorescence whose intensity reaches a peak at a wavelength of λfm is received, the sensitivity of pixels of the R filter 24a is at least greater than the sensitivity of pixels of the G filter 24b and the pixels of the B filter 24c. Note that apart from the present embodiment, when the image pickup section is configured using a single image pickup device, the image pickup section is configured such that the sensitivity of pixels of a color filter for receiving fluorescence is set to be higher than the sensitivity of pixels of the color filter for receiving reflected light other than fluorescence. As in embodiments which will be described later, when the image pickup section is composed of three image pickup devices, pixels of the color filter for receiving fluorescence constitute a first image pickup device for receiving fluorescence and a similar relationship can be obtained if pixels of the color filter for receiving the other two reflected light rays are read as second and third image pickup devices.

As shown in Fig. 4, although pixels of the G filter 24b and pixels of the B filter 24c have the characteristic of maintaining sensitivity even near the wavelength λfm, the pixels other than the pixels of the R filter 24a also receive the fluorescence component but the intensity of the reflected light is by far greater than the intensity of the fluorescence as described above.

As will be described later, when the fluorescence image and the reflected light image (reference image) are displayed together, an image signal value of the reflected light image is by far greater than an image signal value of the fluorescence image, and therefore the image signal of the fluorescence image is adjusted so as to increase its intensity several tens of times relative to the image signal of the reflected light image and the image signal is displayed in color on the color monitor 5. For this reason, the fluorescence image (component) in the image displayed on the color monitor 5 is substantially the image received (imaged) from the pixels of the R filter 24a.

As shown in Fig. 2, the CCD 22 is connected to the video processor 4 via a signal line in the signal cable 27.

The video processor 4 includes a CCD driver 41 and a CCD drive signal generated by the CCD driver 41 is applied to the CCD 22. The CCD 22 generates an image pickup signal by photoelectrically converting an optical image formed on the image pickup surface of the CCD 22 with the application of the CCD drive signal to generate an image pickup signal and outputs the image pickup signal generated. The image pickup signal of the CCD is inputted to an amplifier 43 making up a signal processing circuit 42 in the video processor 4. Note that the signal processing circuit 42 is composed of the amplifier 43 to a D/A conversion section 52 in Fig. 2.

A signal amplified by the amplifier 43 is subjected to a correlated double sampling process by a process circuit 44 to generate an image signal by extracting a signal component from the image pickup signal.

The image signal outputted from the process circuit 44 is converted from an analog to digital image signal in an A/D conversion circuit 45, inputted to an AGC circuit 46 to be adjusted with auto gain, and then inputted to a color separation circuit 47. The color separation circuit 47 separates the image signal into three color signals in accordance with the array of the R filter 24a, the G filter 24b and the B filter 24c in the mosaic filter 24 of the CCD 22 and outputs the three color signals as three image signals.

The color separation circuit 47 outputs color signals of R, G and B as image signals in the normal-light observation mode, and outputs color signals of fluorescence (F), G and B as image signals. Fig. 2 shows the signals as F(R), G and B. The three color-separated image signals are inputted to a white balance/fluorescence balance circuit 48 to be adjusted in white balance or fluorescence balance.

The white balance/fluorescence balance circuit 48 is provided with three amplifiers 48a, 48b and 48c each having a gain variable function and adjusts, in the normal-light observation mode and when an image of an object is picked up as a reference for white color, three gains so that the signal levels of the three R, G and B color signals (image signals) become equal (white balance).

On the other hand, in the fluorescence observation mode, for example, in a fluorescence observation state as a reference, the white balance/fluorescence balance circuit 48 adjusts three gains so as to achieve a fluorescence balance state in which the signal levels of the F, G and B color signals (in other words, image signal of fluorescence and image signals of two reflected light images) become equal.

As described above, since the intensity of fluorescence is much smaller (weaker) than the intensity of reflected light, the gain of the amplifier 48a is adjusted to be at least tens of times the gains of the amplifiers 48b and 48c. Therefore, as described above, even when pixels of the G filter or the like receive near-infrared fluorescence, since the pixels of the R filter increases the gain of the image signal of the received fluorescence to at least several tens of times the gain of the former, the fluorescence received by the former does not affect the fluorescence image of the latter.

Note that a case has been shown where the white balance/fluorescence balance circuit 48 is provided with the three amplifiers 48a, 48b and 48c each gain of which is variable, but, for example, the gain of one amplifier 48b may be fixed and gain adjustment may be performed using the remaining two amplifiers.

The three image signals that have passed through the white balance/fluorescence balance circuit 48 are subjected to gamma correction by a gamma circuit 49 and then inputted to a color emphasis circuit 50 to be subjected to color emphasis. The three image signals outputted from the color emphasis circuit 50 are inputted to a contour emphasis circuit 51 to be subjected to contour emphasis, and then inputted to a D/A conversion section 52.

The D/A conversion section 52 is provided with three D/A conversion circuits 52a, 52b and 52c. The D/A conversion circuits 52a, 52b and 52c each convert a digital input signal to an analog output signal, and the image signal of fluorescence (or R image signal) and the G and B image signals as the three converted image signals are inputted to the R, G and B channels of the color monitor 5.

When the changeover switch 26 is operated, a changeover signal is inputted to a mode determination circuit 53 in the video processor 4. The changeover switch 26 is composed of, for example, an ON/OFF switch, and by determining an H or L level of the changeover signal in accordance with ON/OFF, the mode determination circuit 53 outputs a mode determination signal indicating a determination as to which of an H-level fluorescence observation mode or a L-level normal-light observation mode is selected.

The mode determination circuit 53 outputs the mode determination signal to a control circuit 54 configured to control operation of signal processing by the video processor 4 and a light-adjusting circuit 55 configured to perform light adjustment, and to a light emission control section 34 of the light source apparatus 3.

The control circuit 54 controls the gain adjustment operation of the white balance/fluorescence balance circuit 48 and operations of the color emphasis circuit 50 and the contour emphasis circuit 51 or the like in accordance with the observation mode set through the operation of the changeover switch 26.

Furthermore, the user can variably set, for example, a gain set value (gain adjustment value) or the like when a gain adjustment of the white balance/fluorescence balance circuit 48 is performed from the input section 56 provided with a keyboard or the like, a color emphasis parameter of the color emphasis circuit 50 or a contour emphasis parameter of the contour emphasis circuit 51.

Moreover, for example, the control circuit 54 includes a memory 54a configured to make up a storage section to store (memorize) the gain set value and further store gain set values of the amplifiers 48a to 48c when white balance adjustment is made in the normal-light observation mode and store gain set values of the amplifiers 48a to 48c when fluorescence balance adjustment is made in the fluorescence observation mode. When the observation mode is selected, the control circuit 54 reads gain set values in the selected observation mode from the memory 54a and sets the gains of the amplifiers 48a to 48c to conditions suitable for the observation mode.

The output signals of the white balance/fluorescence balance circuit 48 are inputted to the light-adjusting circuit 55 and the light-adjusting circuit 55 generates a light adjustment signal corresponding to the input signals. The light-adjusting circuit 55 generates, for example, a light adjustment signal in accordance with an amount of deviation from a reference value to bring the image signal closer to the reference value and outputs the light adjustment signal to the light emission control section 34. The light emission control section 34 performs control to increase or decrease emission intensities of the white LED 31a and the excitation LED 31c that emit light in the fluorescence observation mode and emission intensities of the white LEDs 31a and 31b that emit light in the normal-light observation mode in accordance with the light adjustment signal.

Note that instead of adjusting light by increasing or decreasing the emission intensities of the LEDs, a diaphragm may be disposed on the light path that leads to the condensing lens 33 to increase or decrease the amount of aperture of the diaphragm and adjust the amount of illuminating light. When the light amount is adjusted by increasing or decreasing emission intensities of a plurality of LEDs, it may also be possible to increase or decrease emission intensities of the plurality of LEDs while keeping constant a relative intensity ratio of the emission intensities of the plurality of LEDs as in the case of adjusting the amount of illuminating light by increasing or decreasing the amount of aperture of the diaphragm.

According to the present embodiment, it is possible to perform fluorescence observation and normal light observation using one CCD 22 as a single image pickup device.

The fluorescence observation endoscope system 1 according to the present embodiment is provided with the light source apparatus 3 configured to be able to simultaneously emit excitation light as light in a first wavelength band which is radiated onto an ICG as medicine administered to a living body to thereby emit fluorescence, G light which is visible light and as light in a second wavelength band which is different from the light in the first wavelength band and B light which is visible light and as light in a third wavelength band which is different from the light in the first and second wavelength bands, the CCD 22 making up an image pickup section configured to include an image pickup device that simultaneously receives the fluorescence and reflected light of the light in the second and third wavelength bands and the video processor 4 as a signal processing apparatus configured to perform signal processing of generating display images to be displayed in different colors from the image signal of the fluorescence acquired by the image pickup section and the second and third image signals of the reflected light of the light in the second and third wavelength bands. Note that in the present embodiment, the image pickup section is configured using one CCD 22, whereas in a second embodiment which will be described later, the image pickup section is configured using three image pickup devices.

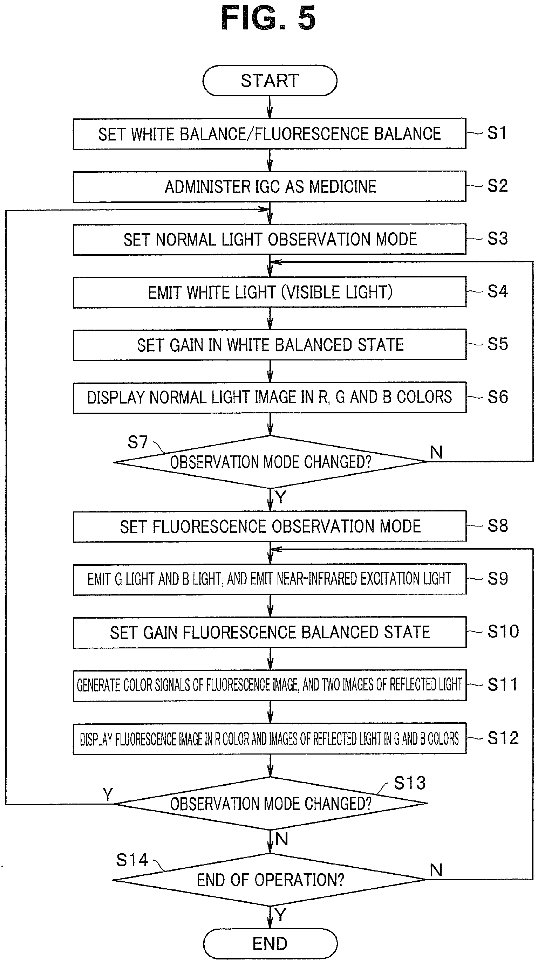

Next, operation of the present embodiment will be described with reference to Fig. 5. Fig. 5 illustrates processing contents in such a case where treatment is performed on the affected area 16 under observation of the endoscope 2.

To perform fluorescence observation, in first step S1, white balance/fluorescence balance is set as an initial setting. Step S1 performs a white balance setting through gain adjustment of the amplifiers 48a to 48c in the white balance/fluorescence balance circuit 48 in the normal-light observation mode using a reference object and a fluorescence balance setting through gain adjustment of the amplifiers 48a to 48c in the fluorescence observation mode. The respective gain set values are stored in the memory 54a. Note that if the white balance setting and the fluorescence balance setting conducted previously are used, a process in next step S2 may be performed without performing the process in step S 1.

In next step S2, the user such as an operator administers medicine (for fluorescence observation) of ICG to a biological tissue in the vicinity of the affected area 16.

In next step S3, the insertion portion 6 of the endoscope 2 is punctured into the abdomen 10 using a trocar which is not shown, and a biological tissue near the affected area 16 to which the ICG is administered is observed by setting the switch of the changeover switch 26 to, for example, the normal-light observation mode. The switch setting of the changeover switch 26 is determined by the mode determination circuit 53 and a mode determination signal is outputted.

When the normal-light observation mode is set, as shown in step 4 (by a mode determination signal), the light source apparatus 3 emits white light (visible light). Furthermore, as shown in step S5 (by a mode determination signal), the control circuit 54 sets the gain of the white balance/fluorescence balance circuit 48 to a gain in a white balanced state.

Furthermore, as shown in step S6, (the signal processing circuit 42 of) the video processor 4 generates R, G and B color signals as image signals under illumination of white light and outputs the generated R, G and B color signals to the R, G and B channels of the color monitor 5 as image signals of normal light images. The color monitor 5 displays the normal light images in R, G and B colors.

Furthermore, as shown in step S7, the mode determination circuit 53 monitors a changeover operation in the observation mode by the changeover switch 26 and determines whether the changeover operation of changing the mode from the normal-light observation mode to the fluorescence observation mode is performed or not. When the changeover operation is not performed, the mode determination circuit 53 maintains the normal-light observation mode and returns to the process in step S4.

On the other hand, when the changeover operation is performed, as shown in step S8, the mode determination circuit 53 sends a mode determination signal whereby the fluorescence observation mode is selected to (the light emission control section 34 of) the light source apparatus 3 and the control circuit 54 of the video processor 4 and sets the light source apparatus 3 and the video processor 4 to the fluorescence observation mode.

That is, as shown in step S9, the light source apparatus 3 sets a state in which the illuminating light in the fluorescence observation mode is emitted, more specifically, a state in which the G light, B light and near-infrared excitation light are emitted. Furthermore, as shown in step S10, the control circuit 54 of the video processor 4 reads the gain set value in the fluorescence observation mode stored in the memory 54a and sets the gains of the amplifiers 48a to 48c of the white balance/fluorescence balance circuit 48 to gains in the fluorescence balanced state. More specifically, the control circuit 54 sets the gain of the amplifier 48a to several tens of times or more the gains of the amplifiers 48b and 48c.

As shown in step S11, (the signal processing circuit 42 of) the video processor 4 performs signal processing on the output signal of the CCD 22 and generates color signals corresponding to the fluorescence image and the two reflected light images (reference light images) respectively. Note that in the case of the present embodiment, the CCD 22 receives (picks up images) reflected light of the G light and the B light by pixels of the G and B filters and receives (picks up images) the fluorescence by pixels of the R filter.

(The signal processing circuit 42 of) the video processor 4 generates an R color signal corresponding to the fluorescence image and G and B color signals corresponding to the reflected light image by the G light and the B light (as image signals) and outputs the color signals to the R, G and B channels of the color monitor 5. Then, as shown in step S12, the color monitor 5 displays the fluorescence image in R color and the two reflected light images (reference light images) in G and B colors.

As described above, the gain of the R color signal corresponding to the fluorescence image is adjusted to at least several tens of times or more of the gains of the G and B color signals of the reflected light images (reference light images) and the R signal is then outputted to the R channel of the color monitor 5, and therefore the color monitor 5 displays the fluorescence image and the reflected light images (reference light images) in color in a well-balanced manner (in the way the operator can easily check both images).

The operator observes the fluorescence image displayed in R color and the reflected light images displayed in two colors of G and B, and can thereby identify regions that emit fluorescence, and the operator observes reflected light images imaged in different wavelength bands and can thereby more easily visually recognize the contours and structure of a biological tissue or an organ in the vicinity of the affected area 16 and differences when a different organ or biological tissue exists based on differences in color tone. For this reason, the operator can more easily recognize the arrangement and shape or the like of biological tissues around the affected area 16 being observed in more detail. Note that in the above description, the R, G and B color signals generated in the video processor 4 are outputted to the R, G and B channels of the color monitor 5 respectively, but the R, G and B color signals may be outputted to the R, B and G channels respectively. That is, the R, G and B color signals may be outputted to any given channels of the color monitor 5 or a selection may be provided so that the R, G and B color signals are outputted to any given channels. In other words, a color signal when fluorescence is received and two color signals when reflected light in two wavelength bands is received, that is, a total of three color signals may be outputted to any given channels of the color monitor 5. Thus, by changing a combination between a color signal and a channel of the color monitor 5, it is possible to improve difference sensitivity (color difference) between reference light and fluorescence. For example, since human eyes have low sensitivity to a blue color, outputting a B signal to a G channel rather than outputting a B signal to a B channel causes the B signal to be displayed in a green color to which the human eyes are more sensitive, making it possible to reduce overlooking of lesions. Note that the present embodiment describes a case where images of the fluorescence and the reflected light are picked up using a single image pickup device, but as in the case of embodiments which will be described later, the present invention is also applicable to a case where the image pickup device that picks up an image of fluorescence is different from the image pickup device that picks up an image of the reflected light.

When the operator observes the fluorescence image and the reflected light image and diagnoses that a region to be removed exists in the affected area 16, the operator takes measures to remove the region using a treatment instrument or the like.

As shown in step S13, the mode determination circuit 53 monitors the changeover operation in the observation mode by the changeover switch 26 to determine whether a changeover operation of changing the fluorescence observation mode to the normal-light observation mode is performed or not.

When the changeover operation is performed, the flow returns to the process in step S3. When the changeover operation is performed, the mode determination circuit 53 sends the mode determination signal whereby the mode is changed to the normal-light observation mode to (the light emission control section 34 of) the light source apparatus 3 and the control circuit 54 of the video processor 4, and the light source apparatus 3 and the video processor 4 are set in the normal-light observation mode.

On the other hand, when the changeover operation is not performed, it is determined whether an end of the observation is instructed or not in step S 14, and if an end of the observation is not instructed, the state of the fluorescence observation mode is maintained and the flow returns to the process in step S9.

According to the present embodiment that operates in this way, the fluorescence image and reflected light images in the two different wavelength bands in the visible region are displayed in different colors, and it is thereby possible to generate images reflecting differences in the contours and color tones between the fluorescence image and the biological tissue. Moreover, it is possible to provide images that help the operator smoothly diagnose and take measures, for example.

Note that in consideration of variations in the characteristic of the wavelength band of the excitation light at the time of manufacturing filters, if the wavelength band of the excitation light is assumed to be 710 nm to 790 nm, the light-shielding range of the excitation light cut filter 25 shown in Fig. 4 may be set to 700 nm to 800 nm so that an interval of 10 nm may be formed between the value of the excitation light at the end on the long wavelength side (more specifically 790 nm) and the value at the end on the short wavelength side in the case of receiving fluorescence (more specifically 800 nm). In Fig. 4, a two-dot dashed line shows a light-shielding characteristic (light-shielding range) of the excitation light cut filter 25 in this case.

Next, a first modification of the first embodiment will be described.

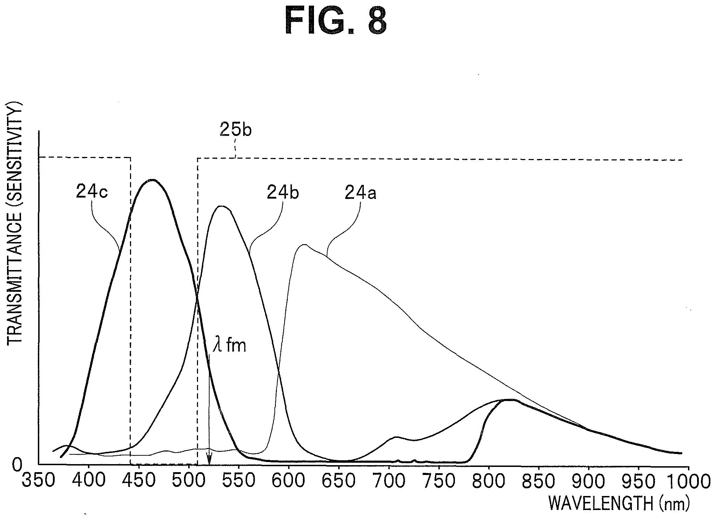

Although a case has been described above where ICG is used as medicine, fluorescein isothiocyanate (abbreviated as "FITC") may also be used. FITC is known to exhibit a fluorescence generation characteristic in which intensity of radiating fluorescence reaches a local maximum at a wavelength of λfm (λfm=521 nm). Fig. 8 shows the wavelength λfm. FITC is also known to exhibit a characteristic in which absorption reaches a local maximum at a wavelength of λex (λex=494 nm) which is closer to the short wavelength side than the wavelength λfm.

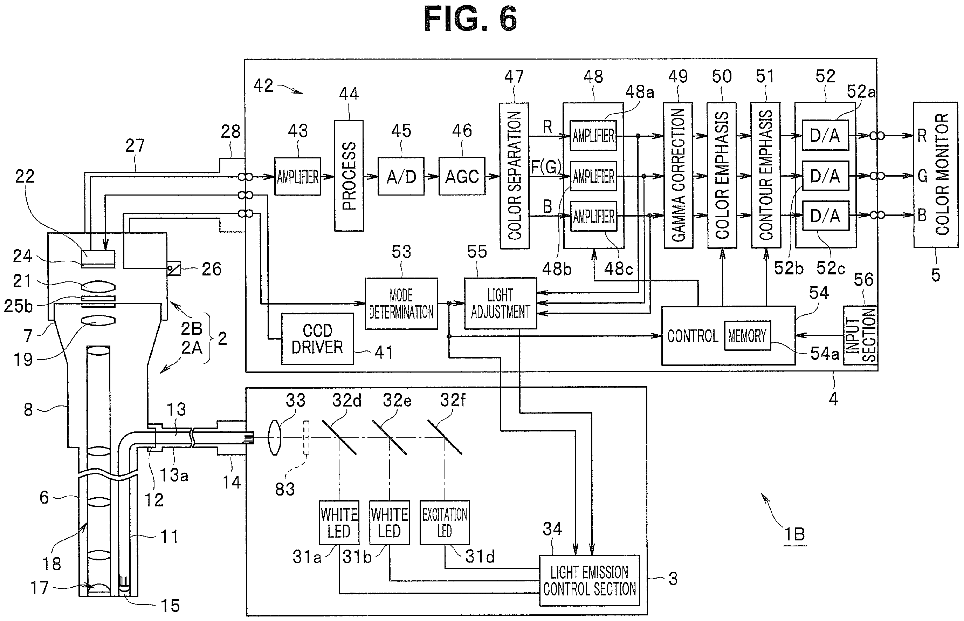

Fig. 6 shows a configuration of a fluorescence observation endoscope system 1B in this case. The fluorescence observation endoscope system 1B shown in Fig. 6 has a configuration in which the dichroic mirrors 32a, 32b and 32c in the light source apparatus 3 in the fluorescence observation endoscope system 1 in Fig. 2 are changed to dichroic mirrors 32d, 32e and 32f respectively and the excitation light source 31 c is changed to an excitation light source 31 d, and the excitation light cut filter 25 in the television camera 2B is substituted by an excitation light cut filter 25b having a different excitation light cut filter characteristic.

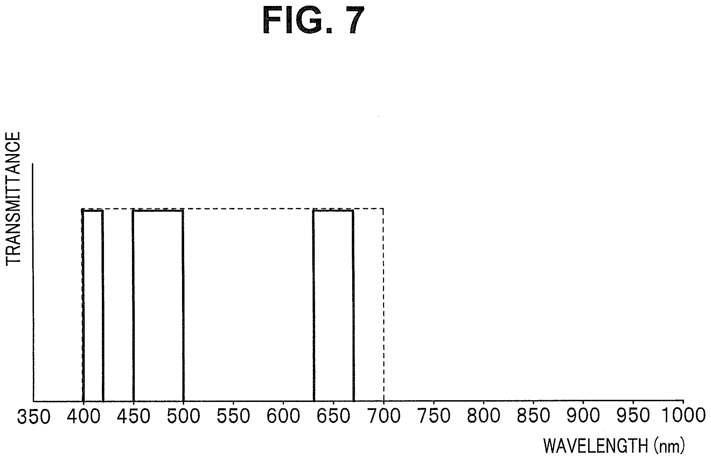

The dichroic mirror 32d selectively reflects light of, for example, 630 nm to 670 nm which is a second wavelength band shown in Fig. 7 and light of, for example, 400 nm to 420 nm which is a third wavelength band and selectively transmits light in other wavelength bands.

Furthermore, the dichroic mirror 32e has a characteristic of substituting the selective transmission characteristic of the dichroic mirror 32d by a reflection characteristic with respect to the incident white light of the excitation light source 31 d composed of, for example, a white LED and selectively transmitting other wavelengths. In other words, when the two white LEDs 31a and 31b are caused to simultaneously emit light, white light in a visible wavelength band of 400 nm to 700 nm is condensed by the condensing lens 33 and made incident upon the light guide 13 as in the case of the first embodiment. In Fig. 7, a dotted line shows a range of the wavelength band of illuminating light emitted from the light source apparatus 3 when the white LEDs 31a and 31 b are caused to simultaneously emit light.

Furthermore, the excitation LED 31c and the dichroic mirror 32c generate excitation light as the light in the first wavelength band. For example, when the excitation LED 31c is assumed to be a white LED, the dichroic mirror 32f selectively reflects the light of 450 nm to 500 nm as the excitation light in the first wavelength band and guides the light to the dichroic mirror 32e side. This excitation light passes through the dichroic mirrors 32e and 32d, is condensed by the condensing lens 33 and made incident upon the light guide 13.

As in substantially the same way as in the case of the first embodiment, the light emission control section 34 performs control so as to cause the white LED 31a and the excitation light source 31d to simultaneously emit light in the fluorescence observation mode and cause the white LEDs 31a and 31 b to simultaneously emit light in the normal observation mode. Note that in the case of the fluorescence observation mode as shown by a dotted line in Fig. 6, an excitation filter 83 or a band limiting filter configured to cut light of, for example, 420 nm to 430 nm including an excitation wavelength at which auto fluorescence is generated may be disposed on the illuminating light path so as to prevent auto fluorescence from mixing into fluorescence caused by a fluorescent agent to be observed.

For the excitation light cut filter 25b, a light-shielding characteristic is set so as to cut light in a wavelength band of the excitation light as shown by a dotted line in Fig. 8. More specifically, a characteristic of light-shielding light of, for example, 440 nm to 510 nm including a margin of 10 nm on the long wavelength side and the short wavelength side respectively (characteristic in which the transmittance becomes substantially 0) is set so as to reliably light-shield the light of 450 nm to 500 nm as the above-described wavelength band of the excitation light. Note that in a case where variations when the filter is created can be reduced to substantially 0, the light-shielding range of the excitation light cut filter 25b may be set to a range of 450 nm to 500 nm.

A solid line in Fig. 8 shows transmission characteristics of the R, G and B filters as in the case of Fig. 4.

In the first embodiment, the R filter is set so as to receive fluorescence, whereas in the present modification, the G filter is set so as to receive fluorescence. For this reason, the video processor 4 processes a signal outputted from pixels of the G filter as an image signal of fluorescence. The color monitor 5 assigns an image signal of fluorescence to the G channel and displays images of reference light assigned to the other R and B channels in color. The rest of the configuration is similar to the configuration of the first embodiment.

Next, operation of the present modification will be described. When a normal-light observation mode is set, the light source apparatus 3 emits illuminating light in a visible wavelength band of 400 nm to 700 nm shown by a dotted line in Fig. 7. The affected area 16 or the like is illuminated with this illuminating light and images of the affected area 16 are picked up by the CCD 22.

In the case of the present modification, reflected light in which part of the wavelength band (more specifically 440 nm to 510 nm) on the long wavelength side in the wavelength band of mainly B light is missing due to the excitation light cut filter 25 is received, but influences of the missing reflected light component are corrected by increasing the gain of the image signal of G during white balance adjustment. When a normal-light observation mode is set, the color monitor 5 displays normal light images in color in substantially the same way as in the first embodiment.

On the other hand, when a fluorescence observation mode is set, the light emission control section 34 of the light source apparatus 3 causes the white LED 31a and the excitation LED 31 c to emit light and the light source apparatus 3 emits illuminating light shown by a solid line in Fig. 7. That is, the light source apparatus 3 emits reference light made up of the light in the R wavelength band (R light) and the light in the B wavelength band (B light) and excitation light in the G wavelength band to the light guide 13 side and the living body is irradiated with such illuminating light.

In this case, pixels of the R filter in the CCD 22 receive reflected light of the R light and pixels of the B filter receive reflected light of the B light. Pixels of the G filter receive fluorescence in the vicinity of the wavelength λfm which reaches a local maximum at 521 nm and the reflected light of the excitation light in this case is sufficiently cut by the excitation light cut filter 25b, not affecting reception of fluorescence.

The video processor 4 operates by reading the pixels of the R filter 24a in the first embodiment as the pixels of the G filter 24b, reading the pixels of the G filter 24b as the pixels of the R filter 24a, reading the R channel as the G channel and reading the G channel as the R channel. The present modification can obtain substantially the same effects as those of the first embodiment.

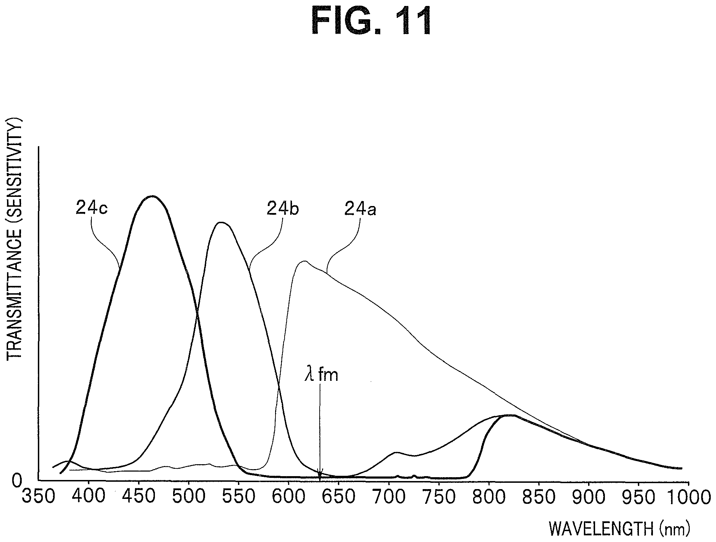

Next, a second modification of the first embodiment will be described. The present modification performs fluorescence observation using 5-aminolevulinic acid (abbreviated as "5-ALA") as the medicine to be administered to a living body. 5-ALA is known to exhibit a fluorescence generation characteristic in which intensity with which fluorescence is emitted reaches a local maximum at a wavelength of λfm (λfm=63 nm). Fig. 11 illustrates the wavelength λfm.

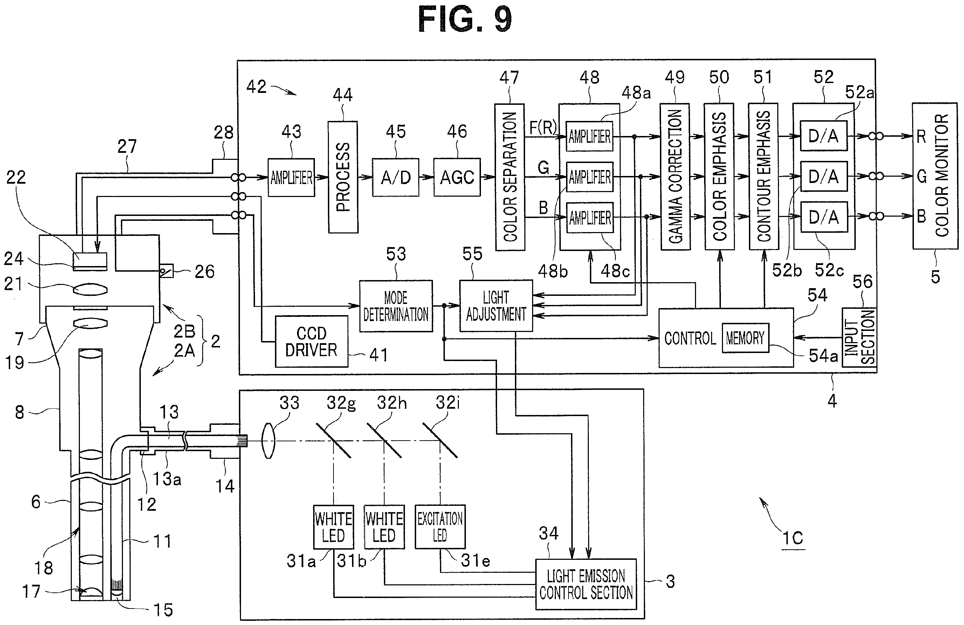

It is also known that the excitation light of a wavelength λex (λex=405 nm) which is a wavelength by far shorter than the wavelength λfm efficiently generates fluorescence. Fig. 9 shows a configuration of a fluorescence observation endoscope system 1C using this medicine.

The present modification adopts a configuration in which the dichroic mirrors 32a, 32b and 32c of the light source apparatus 3 in the fluorescence observation endoscope system 1 shown in Fig. 2 are changed to dichroic mirrors 32g, 32h and 32i, the excitation light source 31 c is changed to an excitation light source 31 e and the excitation light cut filter 25 disposed in the television camera 2B in Fig. 2 is not provided.

The white LED 31a and the dichroic mirror 32g generate reference light of 400 nm to 550 nm. That is, the dichroic mirror 32a in the first embodiment slightly changes the reference light of 400 nm to 570 nm to 400 nm to 550 nm.

The white LED 31b and the dichroic mirror 32h generate R light of 550 nm to 700 nm. That is, the dichroic mirror 32b in the first embodiment slightly changes the characteristic of selectively transmitting the R light of 570 nm to 700 nm to a characteristic of selectively transmitting the R light of 550 nm to 700 nm.

The excitation LED 31e and the dichroic mirror 32i generate excitation light of 380 nm to 400 nm (or 380 nm to 440 nm). The excitation LED 31e is composed of an LED light source that generates light that covers 380 nm to 400 nm (or 380 nm to 440 nm), and the dichroic mirror 32i selectively reflects light in a wavelength band of 380 nm to 400 nm (or 380 nm to 440 nm) and guides the light to the dichroic mirror 32h side.

The light selectively reflected by the dichroic mirror 32i passes through the dichroic mirror 32h, and further at least light of 380 nm to 400 nm passes through the dichroic mirror 32g and the condensing lens 33 causes the light to enter the end face of the light guide 13.

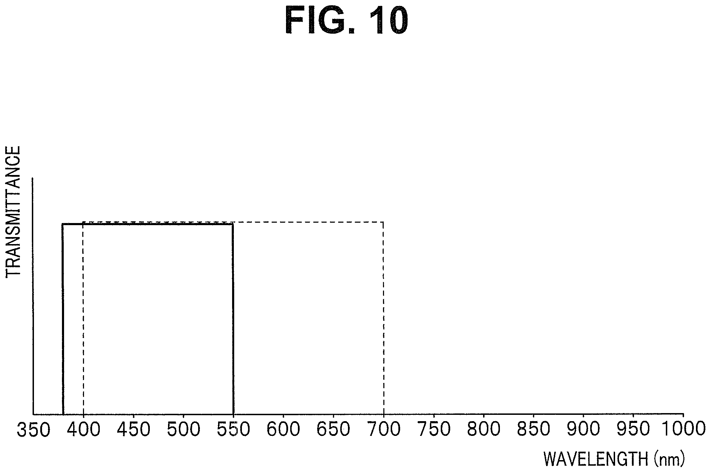

Fig. 10 illustrates a wavelength band of illuminating light emitted by the light source apparatus 3 in the case of the fluorescence observation mode.

In this case, the white LED 31a and the excitation light source 31e simultaneously emit light, and the light source apparatus 3 thereby simultaneously generates the G light and the B light of 400 nm to 550 nm and excitation light of 380 nm to 440 nm and emits the light to the light guide 13 side. Note that the light in the wavelength band of 400 nm to 440 nm is commonly used for illumination with the excitation light and the reference light.

Furthermore, in the case of the normal-light observation mode, as shown by a dotted line in Fig. 10, the light source apparatus 3 emits white light of 400 nm to 700 nm to the light guide 13 side.

The present modification has substantially the same configuration as the first embodiment in the case of the normal-light observation mode, whereas in the fluorescence observation mode, the present modification corresponds to a case where the wavelength band of excitation light in the first embodiment is set to the short wavelength side of R light or vicinity of ultraviolet, and the fluorescence in that case is received using the R filter as in the case of the first embodiment.

In the present modification, since the wavelength band of the excitation light is significantly different from the wavelength band of the fluorescence, it is possible to receive fluorescence without using the excitation light cut filter and without being affected by the excitation light.

For this reason, the CCD 22 as the image pickup section in the present modification receives (picks up images of) light using the R, G and B filters shown by solid lines as shown in Fig. 11 (without using the excitation light cut filter 25 shown by the dotted line in Fig. 4) in both the normal-light observation mode and the fluorescence observation mode. The rest of the configuration is similar to that in Fig. 2.

Next, operation of the present embodiment will be described. In the case of a normal observation mode, operation is substantially the same as that of the first embodiment.

On the other hand, in the case of a fluorescence observation mode, the light source apparatus 3 emits reference light in a wavelength band of 400 nm to 550 nm (G light, B light) and emits excitation light in a wavelength band of 380 nm to 440 nm as illuminating light. In the CCD 22 that makes up the image pickup section, pixels of the G and B filters receive reflected light of the reference light to generate image pickup signals of G and B, and pixels of the R filter receive fluorescence to generate an image pickup signal of the fluorescence.

The video processor 4 performs signal processing on the image pickup signal of the CCD 22 to generate image signals of G and B, and an image signal of the fluorescence, and outputs the signals to the G and B channels and the R channel of the color monitor 5. The color monitor 5 synthesizes the reflected light image in G and B colors and the fluorescence image in R color and displays the synthesized image in color as in the case of the first embodiment.

According to the present modification, it is possible to obtain substantially the same effects as those in the first embodiment without using any excitation light cut filter.

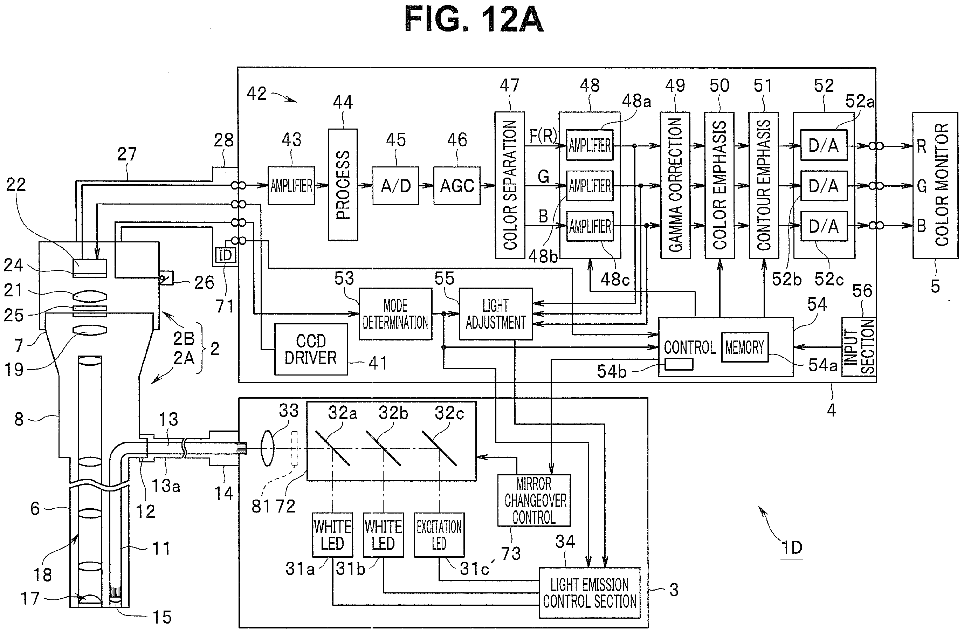

The fluorescence observation endoscope systems 1, 1 B and 1C of the first embodiment adaptable to a case where different kinds of medicine are used have been described above, but it is also possible to adopt a fluorescence observation endoscope system 1D according to a third modification shown in Fig. 12A provided with a video processor as a light source apparatus and a signal processing apparatus adaptable to a case where different kinds of medicine are used.

In the fluorescence observation endoscope system 1D shown in Fig. 12A, for example, the signal connector 28 incorporates an ID generation circuit 71 (simply abbreviated as "ID" in Fig. 12A) configured to generate identification information (abbreviated as "ID") indicating that the endoscopes in Fig. 2, Fig. 6 and Fig. 9 are each specific endoscopes. The ID includes information corresponding to optical characteristics of the image pickup section applicable to each medicine provided for each specific endoscope. Furthermore, when the signal connector 28 is connected, in the video processor 4, for example, the control circuit 54 is provided with an ID identification circuit 54b configured to identify an ID of the endoscope 2. Note that the ID identification circuit 54b may be provided outside the control circuit 54 so as to output an identified ID to the control circuit 54.

The light source apparatus 3 includes a mirror holding apparatus 72 configured to hold three sets of dichroic mirrors (or mirror assembly), each set being composed of three dichroic mirrors so as to be able to emit the illuminating light described in Fig. 2, Fig. 6 and Fig. 9 and a mirror changeover control circuit 73 configured to perform control to dispose one of the three sets of mirror holding apparatuses 72 so that one of these sets is changed in the illuminating light path.

Furthermore, the light source apparatus shown in Fig. 12A uses an excitation light source 31 c' configured to generate light in a visible wavelength band together with the infrared wavelength band as the excitation light source. Note that the three sets of mirror holding apparatuses 72 are configured such that three rotation plates are attached to, for example, the shaft of rotation of a motor, three dichroic mirrors are mounted on the three rotation plates at an interval of rotation angle of 120 degrees respectively to rotate the motor in units of 120 degrees allowing one of the three sets of dichroic mirrors to be disposed on the illuminating light path.

The control circuit 54 controls the mirror changeover control circuit 73 so as to dispose dichroic mirrors corresponding to an endoscope having an identified ID (more specifically, an endoscope provided with the excitation light cut filter 25 or 25b corresponding to the medicine used, or not provided with any excitation light cut filter) on the optical path. That is, the control circuit 54 as control means controls illuminating light emitted from the light source apparatus 3 according to the identified ID and also controls operation of signal processing by (the signal processing circuit 42 of) the video processor 4.

In the example shown in Fig. 12A, the endoscope 2 is an endoscope 2 provided with the excitation light cut filter 25 (provided with an image pickup section corresponding to ICG medicine), and the control circuit 54 controls operation of the mirror holding apparatus 72 so that the dichroic mirrors 32a, 32b and 32c are arranged on the optical path in the light source apparatus 3 in correspondence with the endoscope 2.

When the endoscope 2 (provided with the image pickup section corresponding to FITC medicine) in Fig. 6 instead of the endoscope 2 shown in Fig. 12A is connected to the video processor 4, the control circuit 54 controls operation of the mirror holding apparatus 72 so that the dichroic mirrors 32d, 32e and 32f in Fig. 6 are arranged.

On the other hand, the endoscope 2 (provided with the image pickup section corresponding to 5-ALA medicine) in Fig. 9 instead of the endoscope 2 shown in Fig. 12A is connected to the video processor 4, the control circuit 54 controls operation of the mirror holding apparatus 72 so that the dichroic mirrors 32g, 32h and 32i in Fig. 9 are arranged.

When the endoscope 2 shown in Fig. 12A is connected to the video processor 4, the operation is as described in the first embodiment, providing the effects of the first embodiment. On the other hand, when the endoscope 2 in Fig. 6 is connected to the video processor 4, the operation is as described in the first modification, providing the effects of the first modification. On the other hand, when the endoscope 2 in Fig. 9 is connected to the video processor 4, the operation is as described in the second modification, providing the effects of the second modification. The present modification provides the effects of the first embodiment, the first modification and the second modification, and can also manage a case where fluorescence observation is performed using different kinds of medicine, with a common light source apparatus 3 and a common video processor 4.

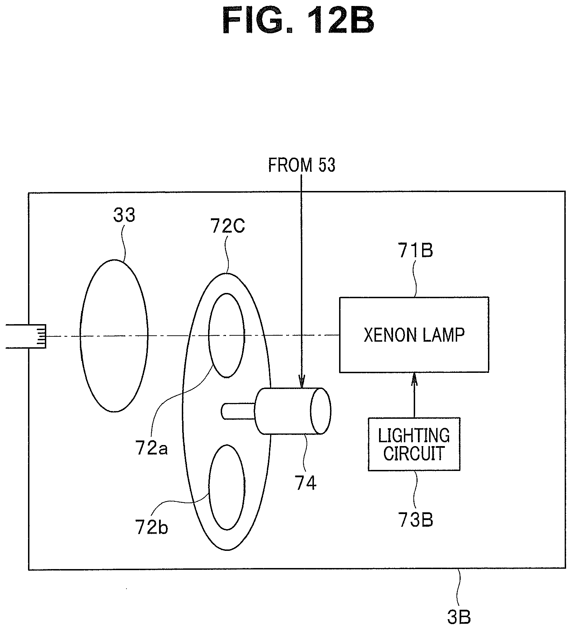

Fig. 12B illustrates a light source apparatus 3B according to a fourth modification of the first embodiment. While the light source apparatus 3 using LEDs is used in the first embodiment, it is also possible to use a light source apparatus 3B constructed using a xenon lamp 71B configured to form a light source and a filter turret 72C as shown in Fig. 12B. Light of the xenon lamp 71 emitted (lighted) by a lighting power supply of a lighting circuit 73 passes through a filter 72a or 72b of the filter turret 72C which is driven to rotate by a motor 74 and the illuminating light is then made incident upon the light guide 13 via the condensing lens 33. The filter turret 72C is provided with the first filter 72a for a normal-light observation mode and a second filter 72b for a fluorescence observation mode in the circumferential direction.

The motor 74 is driven to rotate by a mode changeover signal outputted from the mode determination circuit 53 (of the video processor 4) and configured to dispose one of the filters 72a and 72b of the filter turret 72C on the illuminating light path. The state in Fig. 12B is a state in which a normal-light observation mode is set and a transmission characteristic is set for the first filter 72a so as to transmit the light in the white light wavelength band as shown in Fig. 3B. In contrast, when operation of changing the mode to the fluorescence observation mode is performed, the motor 74 causes the filter turret 72C to rotate and the second filter 72b is disposed on the illuminating light path. The second filter 72b has a transmission characteristic of a bandpass filter set so as to transmit the light in the wavelength band shown in Fig. 3A. Note that in the present modification, even when the observation mode is changed, the xenon lamp 71B as the light source is all the time kept ON (lit) and is not configured to turn ON/OFF light emission.

In the example shown in Fig. 12B, since the light source apparatus 3B is not provided with any function to adjust the amount of light emitted and adjust the amount of illuminating light, the light-adjusting circuit 55 of the video processor 4 is unnecessary. In the configuration in Fig. 12B, a light adjusting signal of the video processor 4 may be inputted to the lighting circuit 73B and the power of the lighting power supply outputted from the lighting circuit 73B may be adjusted based on the light adjusting signals to thereby control the amount of light emitted of the xenon lamp 71B and adjust the amount of illuminating light. Note that the light source apparatus 3B shown in Fig. 12B may also be applicable to a light source apparatus other than the first embodiment using the filter turret 72C which is set so that the second filter 72b has a different transmission characteristic.

### (Second Embodiment)

The aforementioned embodiment and modifications have described the fluorescence observation endoscope system that performs fluorescence observation using the CCD 22 as a single image pickup device, but the image pickup section may be configured using three image pickup devices as will be described below.

Fig. 13 illustrates a fluorescence observation endoscope system 1E according to a second embodiment. The fluorescence observation endoscope system 1E uses an endoscope 2D equipped with a television camera 2C with three built-in CCDs instead of the television camera 2B mounted on the optical endoscope 2A in the fluorescence observation endoscope system 1 shown in Fig. 2 and adopts a video processor 4B configured to perform signal processing on input signals of three channels instead of the video processor 4 configured to perform signal processing on one input signal. Note that the light source apparatus 3 has the same configuration as that of the light source apparatus 3 according to the first embodiment.

The television camera 2C is provided with the excitation light cut filter 25 having the characteristic shown in Fig. 4 opposite to an eyepiece window (shown by a dotted line) and a 3-CCD image pickup section 63 configured to include three dichroic prisms 61 c, 61 a and 61b disposed on an optical path opposite to the image forming lens 21 and CCDs 62c, 62a and 62b respectively mounted on emission surfaces of the dichroic prisms 61c, 61a and 61 b.

Note that the excitation light cut filter 25 is configured to be detachably disposed immediately before the image forming lens 21. For example, if the television camera 2C is mounted after removing the excitation light cut filter 25, the endoscope can be used when 5-ALA is used as medicine. When the excitation light cut filter 25b having the characteristic shown in Fig. 8 is mounted instead of the excitation light cut filter 25 corresponding to the case of the ICG as the fluorescent agent, the endoscope can be used when FITC is used as medicine.

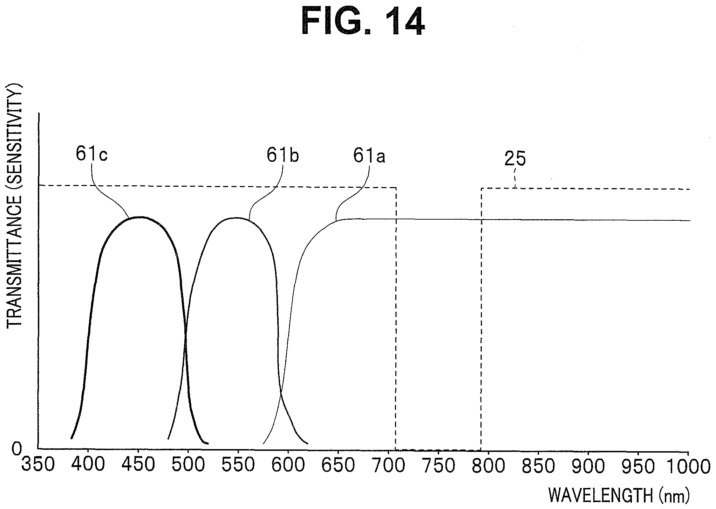

The above-described dichroic prisms 61 a, 61b and 61 c have characteristics as shown in Fig. 14, for example. The dichroic prism 61a is set so as to have a characteristic of transmitting light in the R and infrared wavelength bands (which is received by the CCD 62a disposed on the emission surface), the dichroic prism 61b is set so as to have a characteristic of transmitting light in the G wavelength band (which is received by the CCD 62b disposed on the emission surface), and the dichroic prism 61 c is set so as to have a characteristic of transmitting light in the B wavelength band (which is received by the CCD 62c disposed on the emission surface).

Note that the light that has passed through the image forming lens 21 enters the dichroic prism 61c, and only the B light is selectively reflected on a bonded surface with the dichroic prism 61 a, the reflected B light is further reflected on the incident surface and received by the CCD 62c disposed on the emission surface. Of the light other than the B light that has passed through the bonded surface and entered the dichroic prism 61 a, light other than the G light (R light or infrared) is selectively reflected on a bonded surface with the dichroic prism 61 b, further reflected on a bonded surface with the dichroic prism 61c and (R light or infrared is) received by the CCD 62a disposed on the emission surface.

Furthermore, the G light that has selectively passed through the bonded surface with the dichroic prism 61b is received by the CCD 62b disposed on the emission surface.

In the first embodiment, the image pickup section is configured using the single CCD 22, and therefore the CCD 22 is provided with the mosaic filter 24 including the R, G and B filters, whereas the present embodiment adopts a configuration in which the dichroic prisms 61 a, 61b and 61 c are used instead of using the R, G and B filters and the three CCDs 62c, 62a and 62b are disposed on the emission surface through which the light has passed through the dichroic prisms 61 a, 61b and 61c.

For this reason, the first embodiment may also be configured to use the mosaic filter 24 including R, G and B filters having the characteristic shown in Fig. 14.

Note that when a CCD 62k (k=a, b, c) is defined as an image pickup device configured to receive light that has passed through a dichroic prism 61k, the vertical axis in Fig. 14 represents sensitivity. Furthermore, when fluorescence observation or the like is performed with the characteristic shown in Fig. 14, the dichroic prism 61 a is the only dichroic prism that transmits fluorescence, and only the CCD 62a that receives the light that has passed through the dichroic prism 61 a receives fluorescence in a more favorable condition.

Light rays from the CCDs 62a, 62b and 62c are caused to enter input ends 65a, 65b and 65c of the video processor 4B via signal lines 64a, 64b and 64c. The CCDs 62a, 62b and 62c are connected to the CCD driver 41 via a signal line 64d, and the three CCDs 62a, 62b and 62c are simultaneously driven by a CCD drive signal from the CCD driver 41.

Input signals inputted to the input ends 65a, 65b and 65c are subjected respectively to signal processing by signal processing systems 42a, 42b and 42c respectively and outputted to the R, G and B channels of the color monitor 5. Note that the signal processing systems 42a, 42b and 42c are constructed of an amplifier 43a to an AGC circuit 46a, an amplifier 48a to a D/A conversion circuit 52a, an amplifier 43b to an AGC circuit 46b, an amplifier 48b to a D/A conversion circuit 52b, and an amplifier 43c to an AGC circuit 46c, an amplifier 48c to a D/A conversion circuit 52c respectively as will be described below.

An image pickup signal of the CCD 62a inputted to the input end 65a is outputted to the R channel of the color monitor 5 as an R image signal through the amplifier 43a, the process circuit 44a, the A/D conversion circuit 45a, the AGC circuit 46a, the amplifier 48a in the white balance/fluorescence balance circuit 48, the gamma circuit 49a, the color emphasis circuit 50a, the contour emphasis circuit 51a, and the D/A conversion circuit 52a.

An image pickup signal of the CCD 62B inputted to the input end 65b is outputted to the G channel of the color monitor 5 as a G image signal through the respective circuits where "a" in the amplifier 43a to the AGC circuit 46a, the amplifier 48a to the D/A conversion circuit 52a is substituted by "b" (that is, the amplifier 43b to the AGC circuit 46b, the amplifier 48b to the D/A conversion circuit 52b).

An image pickup signal of the CCD 62B inputted to the input end 65b is outputted to the B channel of the color monitor 5 as a B image signal through the respective circuits where "a" in the amplifier 43a to the AGC circuit 46a, the amplifier 48a to the D/A conversion circuit 52a is substituted by "c" (that is, the amplifier 43c to the AGC circuit 46c, the amplifier 48c to the D/A conversion circuit 52c). The rest of the configuration is substantially the same as the configuration of the fluorescence observation endoscope system 1 in Fig. 2.

In the present embodiment, the light source apparatus 3 can change the light in the first to third wavelength bands corresponding to the fluorescence observation mode to white light corresponding to the normal-light observation mode and emit the white light. The video processor 4B as the signal processing apparatus performs signal processing of generating R, G and B color signals from the signals inputted to the R, G and B channels of the video processor 4B respectively and outputting the signals to the color monitor 5 as the color display apparatus. Note that the signals inputted to the R, G and B channels of the video processor 4B are inputted to the R, G and B channels of the color monitor 5 as they are. In other words, the video processor 4B includes three signal processing systems 42a, 42b and 42c respectively provided with input ends 65a, 65b and 65c for the R, G and B channels whose output ends are connected to the R, G and B channels of the color monitor 5 respectively.

In the case of the present embodiment, (independently of changeover between observation modes or in all observation modes), light from the CCD 62a as the first image pickup device is inputted to the R channel and light from the CCDs 62b and 62c as the second and third image pickup devices are inputted to the G and B channels respectively. In the fluorescence observation mode in particular, as described in the first embodiment (paragraph 0033), the output signals of the first image pickup device, the second and third image pickup devices may be inputted to at least different channels unlike the above-described case. Alternatively, a combination of channels different from the combination of channels (R, G and B channels combined with the output signals of the first to third image pickup devices) set in the normal-light observation mode may be set only in the case of the fluorescence observation mode.

Operation of the present embodiment will become substantially the same operation if pixels that receive the light that has passed through the R filter (that is, pixels of the R filter) are read as the CCD 62a that receives light which has passed through the dichroic prism 61 a, pixels that receive the light that has passed through the G filter (that is, pixels of the G filter) are read as the CCD 62b that receives light which has passed through the dichroic prism 61b, and pixels that receive the light that has passed through the B filter (that is, pixels of the B filter) are read as the CCD 62c that receives light which has passed through the dichroic prism 61 c.

However, in the first embodiment, the color separation circuit 47 in the video processor 4 separates a signal into fluorescence (R), G and B image signals, whereas in the present embodiment, the image pickup section 63 is configured to output fluorescence (R), G and B image pickup signals as three image pickup signals. Thus, the fluorescence (R), G and B image pickup signals are inputted to the three signal processing systems 42a, 42b and 42c of the video processor 4B respectively and the video processor 4B does not perform color separation. The present embodiment has substantially the same effects as those of the first embodiment.

Next, modifications of the second embodiment will be described. The following modifications provide a fluorescence observation endoscope system that reduces influences of auto fluorescence on fluorescence (image) to be observed using a fluorescent agent administered to a living body.

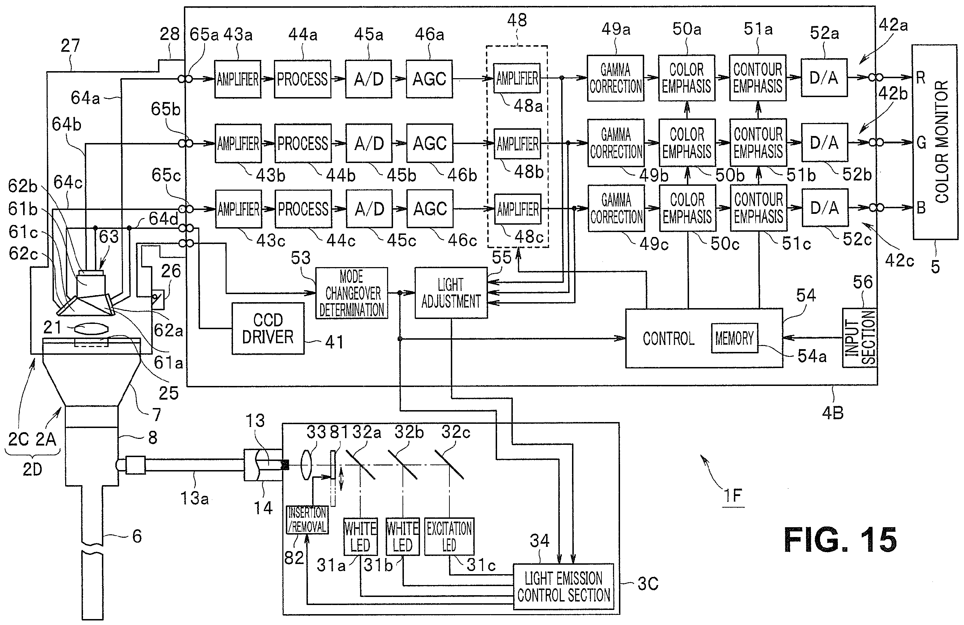

Fig. 15 illustrates an overall configuration of a fluorescence observation endoscope system 1F according to a first modification of the second embodiment. The fluorescence observation endoscope system 1F is provided with an endoscope 2D which is an optical endoscope 2A mounted with a television camera 2C, a light source apparatus 3C, a video processor 4B, and a color monitor 5. The configuration of the endoscope 2D which is the optical endoscope 2A mounted with the television camera 2C has already been described in Fig. 13, and so description thereof is omitted. Moreover, the video processor 4B has the same configuration as that of the video processor 4B described in Fig. 13, and so description thereof is omitted.

The present modification adopts the ICG described in the first embodiment as a fluorescent agent. The endoscope 2D includes the excitation light cut filter 25 described in the first embodiment. In the case of the present modification, an excitation light cut filter having the transmission characteristic shown in Fig. 14 can be adopted as the excitation light cut filter 25.

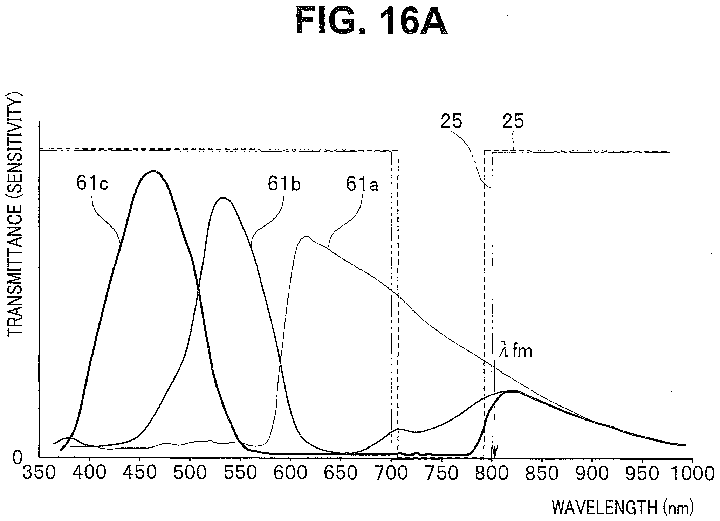

Fig. 14 illustrates the transmission characteristic of the excitation light cut filter 25 and the transmission characteristics of the dichroic prisms 61 a, 61b and 61 c as well, but dichroic prisms 61a, 61b and 61c having characteristics as shown in Fig. 16A may also be adopted. Fig. 16A is a characteristic diagram substantially the same as that in Fig. 4.

The light source apparatus 3C of the present modification shown in Fig. 15 corresponds to the light source apparatus 3 shown in Fig. 2 further provided with an excitation filter 81 configured to limit part of a wavelength band of illuminating light emitted in the fluorescence observation mode. The light emission control section 34 (that makes up a control apparatus or a control section) in the light source apparatus 3C performs control so as to dispose a filter, when the changeover switch 26 selects the fluorescence observation mode, on the illuminating light path as shown by a solid line in Fig. 15 via a filter inserting/removing apparatus 82 and retract the filter, when the normal-light observation mode is selected, from the illuminating light path as shown by a two-dot dashed line. Note that a publicly known apparatus can be adopted for the filter inserting/removing apparatus 82. Alternatively, a rotatable filter turret may be used to configure the filter inserting/removing apparatus 82.

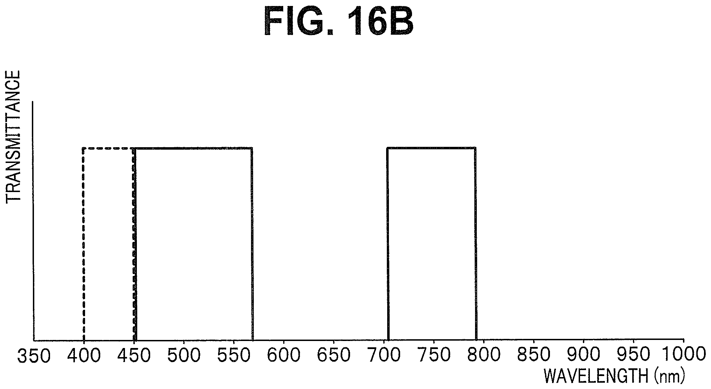

Fig. 16B illustrates a wavelength band (710 to 790 nm) of excitation light as light in a first wavelength band and a wavelength band (450 to 570 nm) of reference light as light in second and third wavelength bands which becomes illuminating light emitted by the light source apparatus 3C in the fluorescence mode using the excitation filter 81. Note that Fig. 16B illustrates a situation in which the light of 450 to 570 nm shown by a dotted line is cut by the excitation filter 81.

In the cases of the first embodiment and the second embodiment, in the fluorescence mode, the light source apparatus 3 emits the light in the wavelength band shown in Fig. 3A to the light guide 13 side, whereas in the present modification, for example, the excitation filter 81 disposed on the illuminating light path immediately before the condensing lens 33 cuts the light in the short wavelength band of 450 nm or less which becomes part of the wavelength band of the reference light (blue wavelength band). The excitation filter 81 has a characteristic of cutting, for example, the light in the short wavelength band of 450 nm or less and transmitting the reference light in a wavelength band longer than 450 nm and the excitation light.

Thus, regarding the excitation light and the reference light that form illuminating light emitted by the light source apparatus 3 in the fluorescence observation mode, the excitation filter 81 according to the present modification of the second embodiment has a function as a band limiting apparatus or a band limiting filter of cutting the light in a short wavelength band of 450 nm or less as part of the wavelength band of the reference light and sufficiently reducing the occurrence of auto fluorescence as will be described below.

The present modification generates an image signal of the R channel which becomes a fluorescence image from an image pickup signal which has passed through the dichroic prism 61a and an image of which is picked up by the CCD 62a, generates image signals of the G and B channels which become two-color reference light images (reflected light images) and outputs the fluorescence image and the two-color reference light images to the R, G and B channels of the color monitor 5 respectively. The color monitor 5 displays red fluorescence image and, green and blue reference light images, superimposed one on another. For this reason, the signal processing systems 42a, 42b and 42c of the present modification (and of the second embodiment) form a superimposed image generation section or a superimposed image generation circuit configured to display a red fluorescence image and, green and blue reference light images, superimposed one on another.

The rest of the configuration of the present modification is similar to that of the second embodiment. Therefore, when the fluorescence observation mode is set in the second embodiment, the operation of the present modification is only different in the operation resulting from the provision of the excitation filter 81.

As will be described below, in the present modification, part of the wavelength band of the reference light adopted in the second embodiment is cut by the excitation filter 81 and it is thereby possible to sufficiently reduce auto fluorescence generated and accurately extract fluorescence (image) using the fluorescent agent to be observed.

In the present modification, a band limiting filter configured to cut light in a short wavelength band of 450 nm or less of reference light is provided as means for reducing influences of auto fluorescence mixing into fluorescence using the fluorescent agent to be observed.

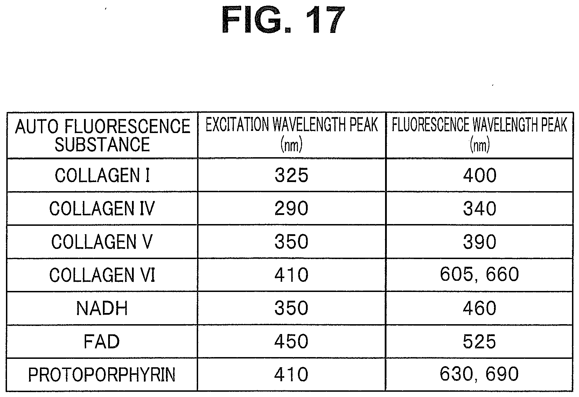

Fig. 17 illustrates a relationship among a plurality of types of auto fluorescence substances contained in a living body, a corresponding (peak of) excitation wavelength and a (peak of) fluorescence wavelength in a table format. Note that the data in Fig. 17 is cited from "Handbook of Biomedical Fluorescence," issued on April, 16, 2003, edited by Mary-Ann Mycek (Editor), Brian W. Pogue (Editor).

For example, collagen I generates auto fluorescence which reaches a peak at 400 nm by excitation light having a peak wavelength of 325 nm. Protoporphyrin generates auto fluorescence which reaches a peak at 630 or 690 nm by excitation light having a peak wavelength of 410 nm. Many of the auto fluorescence substances shown in Fig. 17 are substances in which the center (peak) of a wavelength spectrum of auto fluorescence is included in the green wavelength band. However, collagen VI and protoporphyrin, however weak they may be, generate auto fluorescence in the red wavelength band.

In the present modification, images of fluorescence using ICG as a fluorescent agent are picked up using the dichroic prism 61 a that transmits light in the red and near-infrared wavelength bands.

Since the excitation wavelength of the above-described auto fluorescence substance is located closer to the short wavelength side than 450 nm, by cutting the light in the short wavelength band of 450 nm or less in the reference light, it is possible to effectively reduce the occurrence of auto fluorescence that may possibly be mixed into the fluorescence to be observed. In the present modification, the wavelength band of the reference light is band-limited so as to adopt the reference light from which the light in the short wavelength band of 450 nm or less is cut as shown in Fig. 16B.

The present modification is basically configured so as to adopt illuminating light (reference light) obtained by cutting the light in the short wavelength band of 450 nm or less in the illuminating light (reference light) in the blue wavelength band in the fluorescence observation mode according to the second embodiment.

Thus, as operations and effects of the present modification, it is possible to further reduce the occurrence of auto fluorescence, improve the accuracy of detecting fluorescence using a fluorescent agent or improve contrast of fluorescence images using the fluorescent agent in addition to the operations and effects of the second embodiment. Therefore, the operator can substantially prevent mixture of auto fluorescence from images in the present modification, and can thereby make appropriate diagnosis more easily.

Note that in the present modification, the light in the short wavelength band of 450 nm or less corresponding to the light in the wavelength band at which auto fluorescence is generated is removed from the wavelength band of the reference light or band-limited so that the reference light (as the light of in the second and third wavelength bands) does not include the light in the wavelength band which becomes excitation light that generates auto fluorescence, but only part of the light in the short wavelength band of 450 nm or less may be band-limited.

In the present modification, since images of fluorescence using the fluorescent agent are picked up using an image pickup device set so as to have sensitivity in the red wavelength band and the near-infrared wavelength band, band limitation (on the wavelength band of the reference light) may be performed so as not to include the light in a wavelength band in which auto fluorescence is generated in the wavelength band in which the image pickup device has sensitivity (at which the excitation wavelength reaches a peak). In the case of the auto fluorescence substance in Fig. 17, band limitation may be performed so as to exclude the wavelength band of 400 nm to 420 nm including at least 410 nm (at which the excitation wavelength reaches a peak) from the wavelength band of the reference light.

In other words, regarding the light in the second wavelength band or the light in the third wavelength band (that is, reference light), the light in part of the wavelength band corresponding to the wavelength band which becomes excitation light that generates auto fluorescence may be cut within the wavelength band of fluorescence generated by the fluorescent agent administered to the living body or in the vicinity of the wavelength band of the fluorescence.

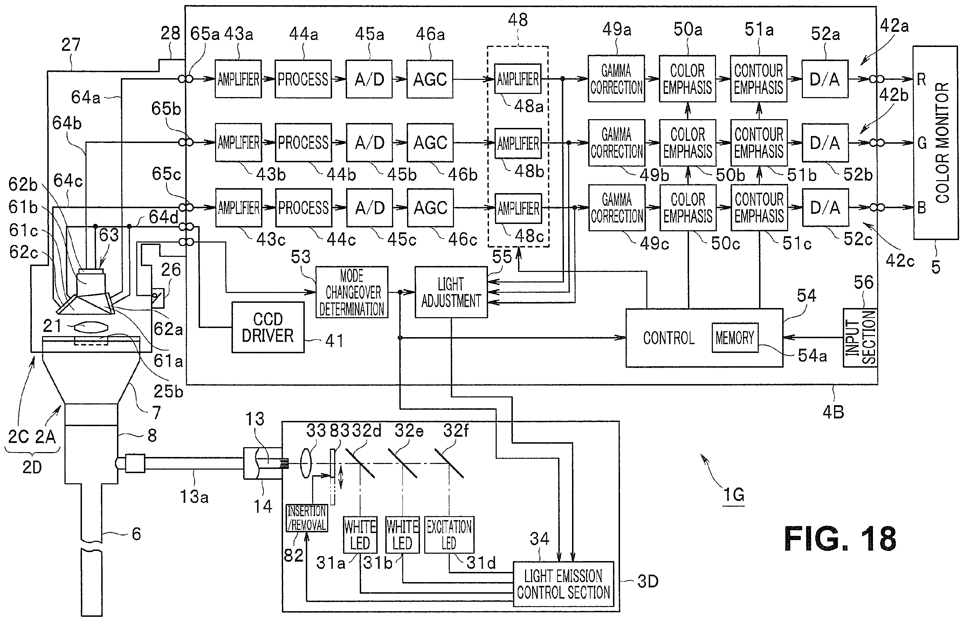

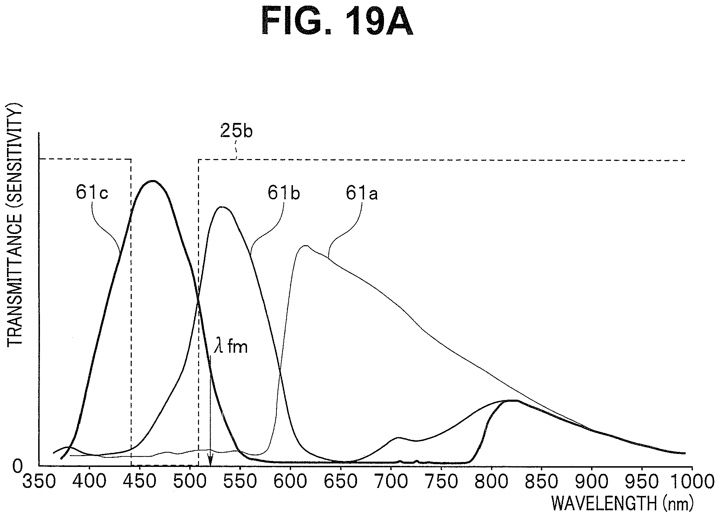

Fig. 18 illustrates an overall configuration of a fluorescence observation endoscope system 1 G according to a second modification of the second embodiment. The fluorescence observation endoscope system 1 G is provided with an endoscope 2D which is an optical endoscope 2A mounted with a television camera 2C, a light source apparatus 3D, the video processor 4B and the color monitor 5. The configuration of the endoscope 2D which is the optical endoscope 2A mounted with the television camera 2C is the same as those shown in Fig. 13 and Fig. 15. However, the present modification adopts FITC as the fluorescent agent. For this reason, the endoscope 2D is provided with an excitation light cut filter 25b having a transmission characteristic shown in Fig. 19A.

The video processor 4B has the same configuration as that in Fig. 13. Note that since the present modification detects fluorescence using FITC and generates an image, the video processor 4B generates an image signal of the G channel which becomes an image signal of fluorescence from an image signal picked up by the CCD 62b through the dichroic prism 61b, generates image signals of the R and B channels which become reference light images, and outputs the fluorescence image and the two-color reference light images to the G, R and B channels of the color monitor 5 respectively.

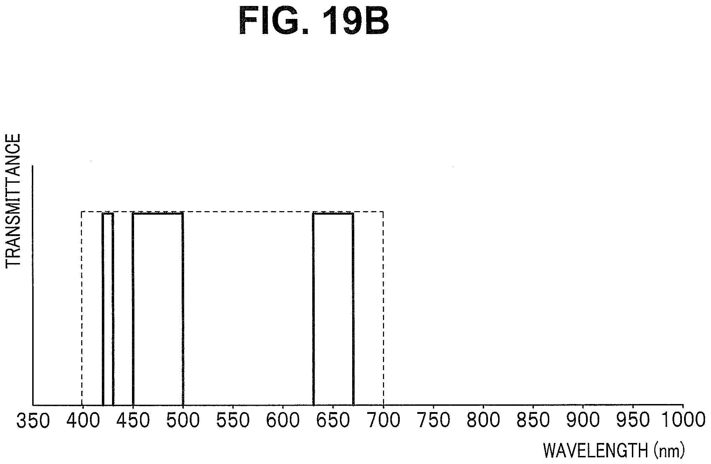

The light source apparatus 3D is configured to cause the light source apparatus 3 shown in Fig. 6 to dispose an excitation filter 83 on the illuminating light path immediately before the condensing lens 33 in the case of the fluorescence observation mode and retract the excitation filter 83 from the illuminating light path in the case of the normal-light observation mode. The light source apparatus 3 in Fig. 6 in which no excitation filter 83 is disposed emits excitation light and reference light having the characteristic shown in Fig. 7 to the light guide 13 side in the case of the fluorescence observation mode. The present modification also adopts the light source apparatus 3D provided with the excitation filter 82, and the light source apparatus 3D emits excitation light as light in a first wavelength band having a characteristic shown in Fig. 19B and reference light as light in second and third wavelength bands to the light guide 13 side in the case of the fluorescence observation mode. The excitation filter 82 band-limits the light having the characteristic shown in Fig. 19 so as to have a characteristic of transmitting only 420 nm to 430 nm of the light on the short wavelength band side equal to or shorter than 450 nm in Fig. 7.

The auto fluorescence substance shown in Fig. 17 does not include (any peak of) the excitation wavelength of 420 nm to 430 nm. For this reason, the present modification adopts the excitation filter 82 having a characteristic of transmitting 420 to 430 nm in the light on the short wavelength side equal to or shorter than 450 nm. Note that the excitation filter 82 has a characteristic oftransmitting light on the long wavelength side longer than 450 nm.

When detecting light in the green wavelength band which becomes light in the wavelength band of fluorescence using a fluorescent agent to be observed, the present modification band-limits part of the wavelength band of the reference light through the excitation filter 82 so as to prevent mixture of fluorescence caused by auto fluorescence into at least the green wavelength band (to reduce auto fluorescence of at least the green wavelength band in which auto fluorescence in the green wavelength band is mainly generated).

Therefore, according to the present modification, it is possible to reduce the auto fluorescence generated, improve the accuracy of detecting fluorescence using a fluorescent agent or improve contrast of a fluorescence image using a fluorescent agent as in the case of the first modification. Therefore, since the operator can substantially reduce mixture of auto fluorescence from image of the present modification, the operator can make an appropriate diagnosis more easily. Note that although a case where the present invention is applied to the second embodiment has been described as the fluorescence observation endoscope system that reduces influences of auto fluorescence, it is obvious that the present invention is also applicable to the first embodiment. For example, in the light source apparatus 3 in Fig. 2, a configuration may be adopted in which the excitation filter 81 shown by a dotted line is disposed in the fluorescence observation mode.

Furthermore, the light source apparatus 3 in Fig. 6 in the fluorescence observation mode may have a configuration in which the excitation filter 83 shown by a dotted line is disposed.

In Fig. 12A, the light source apparatus 3 is configured to selectively dispose three sets of mirror holding apparatuses 72 on the illuminating light path in the fluorescence observation mode so as to emit illuminating light corresponding to the fluorescent agent to be actually used. Furthermore, the light source apparatus 3 may also be configured to perform control so as to dispose the excitation filter 81 or the like that performs band limitation according to the respective fluorescent agents as shown by a dotted line. In this case, the mirror changeover control circuit 73 may be configured to perform changeover so as to dispose the excitation filter corresponding to the fluorescent agent to be actually used. By so doing, it is possible to perform fluorescence observation using a plurality of types of fluorescent agents, sufficiently reduce or exclude mixing of fluorescence of auto fluorescence into fluorescence caused by the fluorescent agent and allow the operator to appropriately make a diagnosis using fluorescence images.

Note that embodiments configured through a partial combination among the aforementioned embodiments also belong to the present invention.

The present application claims priority based on Japanese Patent Application No. 2014-079764, filed in Japan on April 8, 2014, the disclosure of which is incorporated in the specification, claims and drawings of the present application by reference.

## Claims

1. A fluorescence observation endoscope system comprising:
a light source apparatus configured to be able to simultaneously emit light in a first wavelength band which is radiated onto medicine administered to a living body to thereby emit fluorescence, light in a second wavelength band which is visible light having a wavelength band different from the first wavelength band and light in a third wavelength band which is visible light having a wavelength band different from the first and second wavelength bands;
an image pickup section configured to include an image pickup device to simultaneously receive the fluorescence and reflected light of the light in the second and third wavelength bands; and
a signal processing apparatus configured to perform image processing of generating color display images from an image pickup signal of the fluorescence acquired by the image pickup section and second and third image pickup signals acquired from the reflected light of the light in the second and third wavelength bands to be displayed in different colors.

2. The fluorescence observation endoscope system according to claim 1,
wherein the image pickup section comprises only a single image pickup device as the image pickup device.

3. The fluorescence observation endoscope system according to claim 1,
wherein the image pickup section comprises, as the image pickup device, a first image pickup device configured to receive the fluorescence, a second image pickup device configured to receive the reflected light of the light in the second wavelength band and a third image pickup device configured to receive the reflected light of the light in the third wavelength band.

4. The fluorescence observation endoscope system according to claim 3,
wherein the image pickup section comprises, as the first image pickup device, an image pickup device configured to be set such that sensitivity to the light in the fluorescence wavelength band is greater than sensitivity when the light in the second and third wavelength bands is received.

5. The fluorescence observation endoscope system according to claim 2,
wherein the image pickup section comprises, as the single image pickup device, an image pickup device configured to be set such that sensitivity to the light in the fluorescence wavelength band is greater than sensitivity when the light in the second and third wavelength bands is received.

6. The fluorescence observation endoscope system according to claim 2,
wherein the image pickup section comprises, as the single image pickup device, an image pickup device configured to be set so as to receive the fluorescence which is located closer to a long wavelength side than the second and third wavelength bands.

7. The fluorescence observation endoscope system according to claim 3,
wherein the image pickup section comprises, as the second and third image pickup devices, image pickup devices configured to be set such that sensitivity to the fluorescence is lower than sensitivity of the first image pickup device.

8. The fluorescence observation endoscope system according to claim 3,
wherein the image pickup section comprises, as the first image pickup device, an image pickup device configured to be set so as to receive the fluorescence which is located closer to a long wavelength side than the second and third wavelength bands.

9. The fluorescence observation endoscope system according to claim 3,
wherein the light source apparatus can emit white light instead of the light in the first to third wavelength bands,
the signal processing apparatus performs signal processing of generating R, G and B color signals from signals inputted to R, G and B channels respectively and outputting the R, G and B color signals to a color display apparatus, and
an image pickup signal of the first image pickup device is inputted to the R channel and image pickup signals of the second and third image pickup devices are inputted to the G and B channels respectively.

10. The fluorescence observation endoscope system according to claim 2,
wherein the light source apparatus can emit excitation light, the first wavelength band of which varies respectively in accordance with a plurality of types of medicine administered to the living body and illuminating light comprising light in the second and third wavelength bands, and
the signal processing apparatus performs signal processing on the image pickup signal of the fluorescence and the second and third image pickup signals acquired by the image pickup device in correspondence with the illuminating light in accordance with the plurality of types of medicine administered to the living body and generates color display images to be displayed in three different colors.

11. The fluorescence observation endoscope system according to claim 10,
wherein the signal processing apparatus comprises a gain adjusting circuit configured to adjust a signal level corresponding to the image pickup signal of the fluorescence to a gain several tens of times or greater than signal levels of the second and third image pickup signals.

12. The fluorescence observation endoscope system according to claim 11, further comprising a mode changeover switch configured to perform an operation of changing between:
a fluorescence observation mode in which the light source apparatus emits the light in the first to third wavelength bands and the signal processing apparatus performs processing of generating color display images from the image pickup signal of the fluorescence and the second and third image pickup signals outputted from the image pickup section to be displayed in three different colors, and
a normal-light observation mode in which the light source apparatus emits white light instead of the light in the first to third wavelength bands and the signal processing apparatus performs processing of generating color display images for the image pickup signals in three red, green and blue wavelength bands outputted from the image pickup section to be displayed in three different colors.

13. The fluorescence observation endoscope system according to claim 3,
wherein the light source apparatus can emit white light instead of the light in the first to third wavelength bands and the signal processing apparatus performs signal processing of generating R, G and B color signals from signals inputted to R, G and B channels respectively and outputting the R, G and B color signals to a color display apparatus, and
image pickup signals of the first image pickup device, the second and third image pickup devices are inputted to different R, G and B channels respectively.

14. The fluorescence observation endoscope system according to claim 1,
wherein the light source apparatus comprises a band limiting apparatus configured to cut light of part of the wavelength band corresponding to a wavelength band in which an auto fluorescence substance contained in the living body becomes excitation light that generates auto fluorescence from the light in the second wavelength band or the light in the third wavelength band.

15. The fluorescence observation endoscope system according to claim 14,
wherein the band limiting apparatus comprises a band limiting filter configured to cut passage of the light in part of the wavelength band in a short wavelength band of at least 450 nm or less from the light in the second wavelength band or the light in the third wavelength band.

16. The fluorescence observation endoscope system according to claim 14,
wherein the band limiting apparatus cuts the light of part of the wavelength band corresponding to the wavelength band which becomes excitation light that generates the auto fluorescence in the wavelength band of fluorescence generated by the medicine administered to the living body or in a vicinity of the wavelength band of the fluorescence from the light in the second wavelength band or the light in the third wavelength band.

17. The fluorescence observation endoscope system according to claim 12,
wherein the light source apparatus further comprises:
a band limiting apparatus configured to cut part of the wavelength band corresponding to the wavelength band in which an auto fluorescence substance contained in the living body becomes excitation light that generates auto fluorescence from the light in the second wavelength band or the light in the third wavelength band; and
a control apparatus configured to perform control to dispose the band limiting apparatus on an illuminating light path when the mode changeover switch selects the fluorescence observation mode and retract the band limiting apparatus from the illuminating light path when the mode changeover switch selects the normal-light observation mode.

18. The fluorescence observation endoscope system according to claim 1,
wherein the light source apparatus can selectively emit first excitation light or second excitation light having different wavelength bands to be excited in correspondence with first medicine and second medicine of different types as the medicine to be administered to the living body as the light in the first wavelength band, and
the light source apparatus comprises a band limiting apparatus configured to cut part of the wavelength band corresponding to the wavelength band which becomes excitation light that generates the auto fluorescence generated by an auto fluorescence substance contained in the living body in the wavelength band of the fluorescence generated by the first medicine or the second medicine actually administered to the living body or in a vicinity of the wavelength band from the light in the second wavelength band or the light in the third wavelength band.
